# EUROPEAN PATENT APPLICATION

(11) **EP 4 212 633 A1**
(43) Date of publication of application: **19.07.2023**
(21) Application number: 22151440.9
(22) Date of filing: 13.01.2022
(51) Int. Cl.: C12Q 1/6888, G01N 33/50, A61K 31/33

(54) **MOLECULES TARGETING VIRAL RNA AND/OR VIRAL RNA-PROTEIN COMPLEXES FOR USE IN THE TREATMENT OF VIRAL INFECTIONS, IN PARTICULAR COVID-19**

(71) Applicant: Johann-Wolfgang-Goethe-Universität Frankfurt am Main, 60323 Frankfurt am Main (DE); Technische Universität Darmstadt, 64289 Darmstadt (DE)
(72) Inventor: Schwalbe, Harald, 61449 Steinbach/Ts (DE); Schlundt, Andreas, 61352 Bad Homburg v. d. H (DE); Göbel, Michael, 65779 Kelkheim (DE); Ciesek, Sandra, 60322 Frankfurt am Main (DE); Weigand, Julia, 64293 Darmstadt (DE); Peter, Stephen, 63110 Rodgau (DE); Ferner, Jan, 60439 Frankfurt am Main (DE); Wacker, Anna, 61206 Wöllstadt (DE); Richter, Christian, 60438 Frankfurt am Main (DE); Sreeramulu, Sridhar, 60439 Frankfurt am Main (DE); Ceylan, Betül, 63654 Büdingen (DE); Adam, Jennifer, 60433 Frankfurt am Main (DE); Töws, Sabrina, 60437 Frankfurt am Main (DE); Scheffer, Ute, 65830 Kriftel (DE)
(74) Representative: Krauss, Jan

(57) **Abstract**

The present invention relates to a method for identifying a pharmaceutically active compound that modulates the function of a frame-shift pseudoknot element and/or modulates the function of a translation initiation-RNA element of an RNA virus in a mammalian cell. Furthermore, the present invention relates to a method to identify a second and further generation candidate compound with improved modulating properties, a screening system for identifying the pharmaceutically active compound, as well as compounds as identified, and methods of treating or preventing a viral infection caused by an RNA virus in a mammalian cell.

## Description

The present invention relates to a method for identifying a pharmaceutically active compound that modulates the function of a frame-shift pseudoknot element and/or modulates the function of a translation initiation-RNA element of an RNA virus in a mammalian cell. Furthermore, the present invention relates to a method to identify a second and further generation candidate compound with improved modulating properties, a screening system for identifying the pharmaceutically active compound, as well as compounds as identified, and methods of treating or preventing a viral infection caused by an RNA virus in a mammalian cell.

### Background of the invention

Betacoronaviruses (β-CoVs or Beta-CoVs) are one of four genera of coronaviruses of the subfamily *Orthocoronavirinae* in the family *Coronaviridae,* of the order *Nidovirales.* They are enveloped, single-stranded positive-strand RNA viruses of zoonotic origin.

Recently, a novel coronavirus emerged in the Chinese city of Wuhan in December 2019. After human coronavirus 229E (HCoV-229E) (classified in the genus *Alphacoronavirus*) and HCoV-OC43 (*Betacoronavirus* lineage 2a member) described in the 1960s, SARS-CoV-(*Betacoronavirus* lineage 2b member) that emerged in March 2003, HCoV-NL63 (*Alphacoronavirus* lineage 1b member) described in 2004, HCoV-HKU1 (B*etacoronavirus* lineage 2a member) discovered in 2005, and finally MERS-CoV that emerged in 2012 (classified in *Betacoronavirus* lineage 2c), the novel coronavirus is the seventh human coronavirus described to date as being responsible for respiratory infection. Evidence was rapidly reported that patients were suffering from an infection with a novel *Betacoronavirus* tentatively named 2019 novel coronavirus (2019-nCoV). Despite drastic containment measures, the spread of 2019-nCoV, now officially known as severe acute respiratory syndrome coronavirus 2 (SARS-CoV-2), is ongoing in 2022. Phylogenetic analysis of this virus indicated that it is different (~80% nucleotide identity) but related to SARS-CoV.

Although numerous pharmacological interventions are under clinical investigation, clear efficacy has not yet been determined for any drug. Several drugs have been tested against COVID-19 in vitro, such as, for example remdesivir, chloroquine or hydrochloroquine (Wang et al., 2020), and some showed an activity in a single-digit micromolar concentration. Ribavirin, remdesivir or favipavir exhibit an antiviral activity only in the two- or three-digit micromolar range (Wang et al., 2020). Recently, the α-ketoamide-Inhibitor 13b was described as an anti-SARS-CoV-2 agent, also showing an antiviral activity in the low micromolar range (Zhang et al., 2020). Although some promising results were obtained with chloroquine (Millán-Oñate et al., 2020), a phase IIb-study identified heart arrhythmia as side-effects at higher concentrations (Borba et al., 2020). Similarly, the drug lopinavir was found to have no effect in clinical tests (Cao et al., 2020).

A wide range of RNA viruses use programmed -1 ribosomal translational frameshifting for the production of viral fusion proteins. All coronaviruses utilize the programmed -1 ribosomal frameshift (-1 PRF) to control the relative expression of their proteins. Previous analyses of SARS-CoV have revealed that the virus employs a structurally unique three-stemmed mRNA pseudoknot that stimulates high -1 PRF rates and that it also harbors a -1 PRF attenuation element.

Plant et al. (in: Plant EP, Perez-Alvarado GC, Jacobs JL, Mukhopadhyay B, Hennig M, Dinman JD. A three-stemmed mRNA pseudoknot in the SARS coronavirus frameshift signal. PLoS Biol. 2005 Jun;3(6):e172. doi: 10.1371/journal.pbio.0030172. Epub 2005 May 17. PMID: 15884978; PMCID: PMC1110908) identified a new RNA structural motif that is capable of promoting efficient programmed ribosomal frameshifting. The high degree of conservation of three-stemmed mRNA pseudoknot structures among the coronaviruses suggests that this presents a novel target for antiviral therapeutics.

In order to discover ligands for the SARS-pseudoknot by virtual screening, Park et al. (Park SJ, Kim YG, Park HJ. Identification of RNA pseudoknot-binding ligand that inhibits the -1 ribosomal frameshifting of SARS-coronavirus by structure-based virtual screening. J Am Chem Soc. 2011 Jul 6;133(26):10094-100. doi: 10.1021/ja1098325. Epub 2011 Jun 14. Erratum in: J Am Chem Soc. 2011 Sep 7;133(35): 14150. PMID: 21591761) constructed a 3D structural model of the SARS-pseudoknot and conducted a computational screening of the chemical database. After virtual screening of about 80,000 compounds against the SARS-pseudoknot structure, high-ranked compounds were selected and their activities were examined by in vitro and cell-based -1 RF assay. They identified a novel ligand 43 that inhibits the -1 PRF of SARS-CoV. This antiframeshift agent is described as an interesting lead for the design of novel antiviral agents against SARS-CoV.

Kelly et al. (Kelly JA, Olson AN, Neupane K, et al. Structural and functional conservation of the programmed -1 ribosomal frameshift signal of SARS-CoV-2. Preprint. bioRxiv. 2020;2020.03.13.991083. Published 2020 Jun 15. doi:10.1101/2020.03.13.991083) also disclose that altering -1 PRF activity impairs virus replication, suggesting that this activity may be therapeutically targeted. They comparatively analyzed the SARS-CoV and SARS-CoV-2 frameshift signals. Structural and functional analyses revealed that both elements promote similar -1 PRF rates and that silent coding mutations in the slippery sites and in all three stems of the pseudoknot strongly ablate -1 PRF activity. Small-angle X-ray scattering analyses indicated that the pseudoknots in SARS-CoV and SARS-CoV-2 have the same conformation. Finally, a small molecule previously shown to bind the SARS-CoV pseudoknot and inhibit -1 PRF was similarly effective against -1 PRF in SARS-CoV-2, suggesting that such frameshift inhibitors may be promising lead compounds to combat the current COVID-19 pandemic.

WO2010/019243 discloses compounds that modulate ribosomal frameshifting and nucleic acid constructs for use in methods for identifying or validation such compounds. The document relates to the use of nucleic acid constructs to identify or validate compounds capable of modulating the efficiency of programmed ribosomal frameshifting and the use of said compounds to inhibit the replication or infectivity of viruses that employ programmed ribosomal frameshifting.

US 20080207597 discloses a homopiperazine compound for inhibiting ribosomal frameshifting by binding to an RNA pseudoknot structure of SARS coronavirus. Provided is a pharmaceutical composition for inhibiting synthesis of protein induced by -1 frameshifting by binding to an RNA pseudoknot structure specifically existing in SARS coronavirus. The composition includes a therapeutically effective amount of homopiperazine compound of the chemical formula 1 or a pharmaceutically accepted salt thereof, and a pharmaceutically accepted carrier or excipient.

Schubert K, et al. (in: SARS-CoV-2 Nsp1 binds the ribosomal mRNA channel to inhibit translation. Nat Struct Mol Biol. 2020 Oct;27(10):959-966. doi: 10.1038/s41594-020-0511-8. Epub 2020 Sep 9) describe that SARS-CoV-2 non-structural protein 1 (Nsp1), also referred to as the host shutoff factor, suppresses host innate immune functions. By combining cryo-electron microscopy and biochemistry, they show that SARS-CoV-2 Nsp1 binds to the human 40S subunit in ribosomal complexes, including the 43S pre-initiation complex and the non-translating 80S ribosome. The protein inserts its C-terminal domain into the mRNA channel, where it interferes with mRNA binding. They observe translation inhibition in the presence of Nsp1 in an in vitro translation system and in human cells. They further show that the full-length 5' untranslated region of the genomic viral mRNA stimulates translation in vitro, suggesting that SARS-CoV-2 combines global inhibition of translation by Nsp1 with efficient translation of the viral mRNA to allow expression of viral genes.

Green L and Goff SP (in: Translational readthrough-promoting drugs enhance pseudoknot-mediated suppression of the stop codon at the Moloney murine leukemia virus gag-pol junction. J. Gen. Virol. 2015;96(11):3411-3421. doi:10.1099/jgv.0.000284) disclose the effects of readthrough-promoting drugs - aminoglycoside antibiotics and the small molecule ataluren - on the efficiency of readthrough of the stop codon in the context of the MoMLV genome. The resulting elevated gag-pol readthrough had deleterious effects on virus replication.

Alterations in programmed ribosomal frameshifting efficiencies can have a pronounced negative effect on viral production. Thus, ribosomal frameshifting is a compelling, unexploited target for novel antiviral agents (Dinman, et al., 1998, Trends Biotechnol. 16:190-196).

It is therefore an object of the present invention to provide effective anti-frameshift agents that can be used for the prevention and treatment of viral infection, in particular SARS-CoV-2 infection. Other objects and advantages will become apparent to the person of skill when studying the present description of the present invention.

In order to solve the above problem, the present invention relates to compounds that modulate ribosomal frameshifting elements and/or modulate the function of a translation initiation-RNA element, as well as methods for identifying or validating such compounds. In particular, the present invention relates to antiviral compounds capable of modulating the efficiency of viral programmed ribosomal frameshifting and/or modulate the function of a translation initiation-RNA element, methods for identifying or validating such compounds and methods for treating viral infections using such compounds.

The present invention was devised to overcome the limitation of conventional antiviral drugs by targeting an RNA pseudoknot structure and/or a translation initiation-RNA element of SARS coronavirus, and is directed to provide antiviral compounds that have excellent activity in inhibiting viability of an RNA virus, such as the SARS coronavirus, and pharmaceutical compositions including the antiviral compound.

Other objects and advantages of the present invention can be understood by the following description, and become apparent with reference to the embodiments of the present invention. Also, it is obvious to those skilled in the art to which the present invention pertains that the objects and advantages of the present invention can be realized by the means as claimed and combinations thereof.

In a first aspect of the present invention, the above object is solved by a method for identifying a pharmaceutically active compound that modulates the function of a frame-shift pseudoknot element and/or modulates the function of a translation initiation-RNA element of an RNA virus in a mammalian cell, comprising a) contacting the frame-shift pseudoknot element and/or the translation initiation-RNA element of said RNA virus or a functional fragment thereof with at least one candidate compound, and b) detecting a binding of the at least one candidate compound to the frame-shift pseudoknot element and/or the translation initiation-RNA element of said RNA virus or a functional fragment thereof, wherein a binding of said candidate compound is indicative for a pharmaceutically active compound.

Preferred is the method according to the present invention, further comprising the step of detection a modulation of the translation of one or more viral proteins of said RNA virus compared to the translation in the absence of said at least one candidate compound, wherein a modulation of the translation of the said at least one or more viral protein is indicative for a pharmaceutically active compound.

The invention is based, in part, on the Applicants' discovery that frame-shift pseudoknot elements and/or translation initiation-RNA elements in RNA viruses are conserved and that in addition to viral proteins, structured viral RNA elements represent a potent alternative as drug targets. The present approach is based on inhibiting two major steps in the viral life cycle, namely to inhibit the viral hijacking of translation (5_SL1 element), and/or to inhibit the stage 1 to stage 2 transition (frameshift element (PK)).

Bhatt et al. (in: Bhatt PR, Scaiola A, Loughran G, Leibundgut M, Kratzel A, Meurs R, Dreos R, O'Connor KM, McMillan A, Bode JW, Thiel V, Gatfield D, Atkins JF, Ban N. Structural basis of ribosomal frameshifting during translation of the SARS-CoV-2 RNA genome. Science. 2021 Jun 18;372(6548): 1306-1313. doi: 10.1126/science.abf3546. Epub 2021 May 13. PMID: 34029205; PMCID: PMC8168617) discloses that programmed ribosomal frameshifting is a key event during translation of the severe acute respiratory syndrome coronavirus 2 (SARS-CoV-2) RNA genome that allows synthesis of the viral RNA-dependent RNA polymerase and downstream proteins. They present the cryo-electron microscopy structure of a translating mammalian ribosome primed for frameshifting on the viral RNA. The viral RNA adopts a pseudoknot structure that lodges at the entry to the ribosomal messenger RNA (mRNA) channel to generate tension in the mRNA and promote frameshifting, whereas the nascent viral polyprotein forms distinct interactions with the ribosomal tunnel. Biochemical experiments validate the structural observations and reveal mechanistic and regulatory features that influence frameshifting efficiency. Finally, they compare compounds previously shown to reduce frameshifting with respect to their ability to inhibit SARS-CoV-2 replication, establishing coronavirus frameshifting as a target for antiviral intervention. They also disclose that pseudoknots regulate the expression of ppla and pp1a/b via -1 ribosomal frameshifting.

Wacker and Weigand (in: Secondary structure determination of conserved SARS-CoV-2 RNA elements by NMR spectroscopy. Nucleic Acids Res. 2020 Dec 16;48(22):12415-12435. doi: 10.1093/nar/gkaa1013. Erratum in: Nucleic Acids Res. 2021 Jul 9;49(12):7204-7205. PMID: 33167030; PMCID: PMC7736788) disclose that the current pandemic situation caused by the Betacoronavirus SARS-CoV-2 highlights the need for coordinated research to combat COVID-19. A particularly important aspect is the development of medication. In addition to viral proteins, structured RNA elements represent a potent alternative as drug targets. The search for drugs that target RNA requires their high-resolution structural characterization. Using nuclear magnetic resonance (NMR) spectroscopy, a worldwide consortium of NMR researchers aims to characterize potential RNA drug targets of SARS-CoV-2. They report the characterization of 15 conserved RNA elements located at the 5' end, the ribosomal frameshift segment and the 3'-untranslated region (3'-UTR) of the SARS-CoV-2 genome, their large-scale production and NMR-based secondary structure determination. The NMR data are corroborated with secondary structure probing by DMS footprinting experiments. The close agreement of NMR secondary structure determination of isolated RNA elements with DMS footprinting and NMR performed on larger RNA regions shows that the secondary structure elements fold independently. The NMR data reported provide the basis for NMR investigations of RNA function, RNA interactions with viral and host proteins and screening campaigns to identify potential RNA binders for pharmaceutical intervention. Thus, a respective RNA biochemistry and NMR spectroscopy was established.

Sun et al. (in: Sun L, Li P, Ju X, et al. In vivo structural characterization of the SARS-CoV-2 RNA genome identifies host proteins vulnerable to repurposed drugs. Cell. 2021;184(7):1865-1883.e20. doi:10.1016/j.cell.2021.02.008) disclose that severe acute respiratory syndrome coronavirus 2 (SARS-CoV-2) is the cause of the ongoing coronavirus disease 2019 (COVID-19) pandemic. Understanding of the RNA virus and its interactions with host proteins could improve therapeutic interventions for COVID-19. By using icSHAPE, they determined the structural landscape of SARS-CoV-2 RNA in infected human cells and from refolded RNAs, as well as the regulatory untranslated regions of SARS-CoV-2 and six other coronaviruses. They validated several structural elements predicted *in silico* and discovered structural features that affect the translation and abundance of subgenomic viral RNAs in cells. The structural data informed a deep-learning tool to predict 42 host proteins that bind to SARS-CoV-2 RNA. Strikingly, antisense oligonucleotides targeting the structural elements and FDA-approved drugs inhibiting the SARS-CoV-2 RNA binding proteins dramatically reduced SARS-CoV-2 infection in cells derived from human liver and lung tumors. Their findings thus shed light on coronavirus and reveal multiple candidate therapeutics for COVID-19 treatment.

In general, the invention is based on the following steps:
a) If not already known, analysis of genomic data of RNA-viruses, in particular betacoronaviruses, for identifying and/or predicting of stable and conserved RNA-structural target elements suitable for a modulation/inhibition.
b) Tailored preparation of these target elements, through rapid high-purity chemical or biological production, and introduction into optimized methods for a biophysical characterization, in particular using NMR-spectroscopy.
c) Parallel determination of such RNA structures as well as - optionally - respective RNA-protein-complexes as target structures that provides those regions that are suitable to be addressed with modulators/inhibitors.
d) Parallel screening of compound libraries (in particular small molecule libraries) for their binding to the RNA-structural target elements, and validation of the results using NMR-spectroscopy (including purity, solubility, stability).
e) Analyses, prioritization of the best binding substances and quantification of their binding properties (affinity, location of binding at the RNA-target structure as well as specificity) by means of NMR-spectroscopic methods and bioinformatics methods.
f) If required, cheminformatic or other methods for identifying of 2^{nd} generation (or further) modulators/inhibitors on the basis of the analysis of initial binding.
g) If f) was performed, experimental validation of the improved properties of these 2^{nd} generation (or further) modulators/inhibitors.
h) Optionally, development of diversity-oriented synthesis of modulators/inhibitors having sub-micromolecular affinity, establishing of these syntheses.
i) Optionally, development of in-vitro and cellular assays for a characterization of the modulators/inhibitors for RNA- or RNA-protein-targets, respectively.
j) Optionally, experimental validation of the improved modulators/inhibitors with respect to their antiviral propagation.

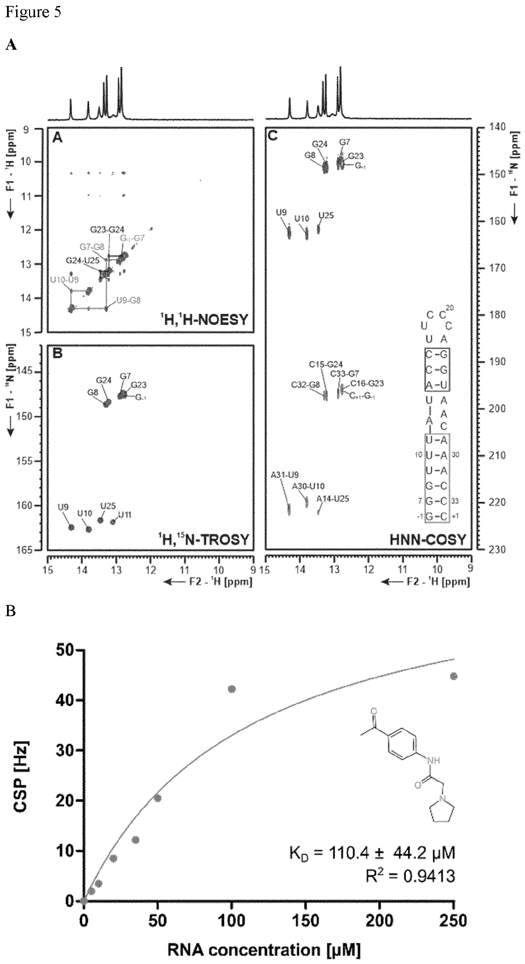

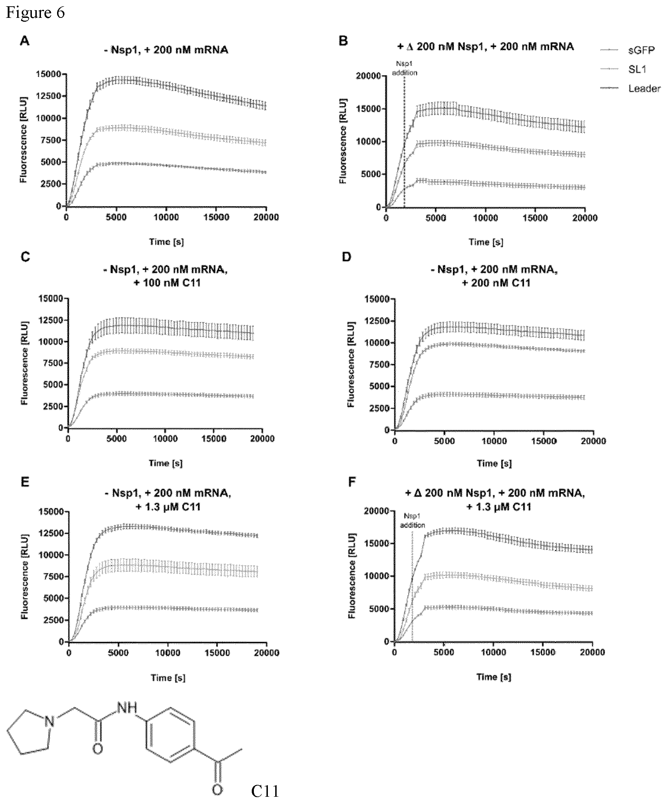

The present invention is exemplified by three prototype-modulators/inhibitors (D01, D01+DAP, and D05), that target the pseudoknot-frameshift element of SARS-CoV-2. The present invention is further exemplified by two prototype-modulators/inhibitors (C11 and C12) that target the translation initiation-RNA 5_SL1 element of SARS-CoV-2 (Figure 5A and B and Figure 6).

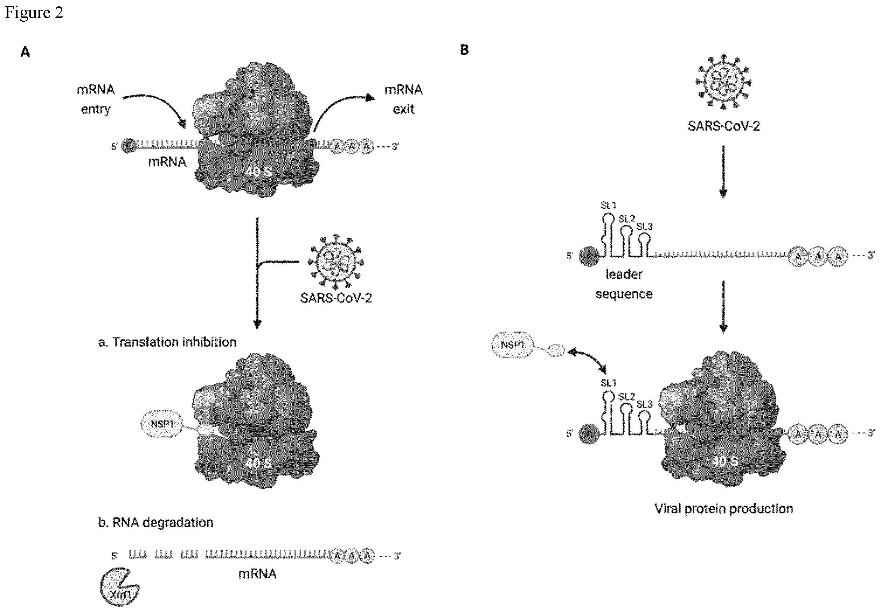

The translation initiation-RNA 5_SL1 element of SARS-CoV-2 is a 5'-terminal structural element in the SARS-CoV-2 RNA genome. The sequence is conserved among betacoronaviruses, comprises nucleotides 7-33 of the genome of SARS-CoV-2, a *cis*-regulatory element, and is GC-stabilized. Nsp1 is encoded at the 5' end of ORF1a, is the first viral protein to be produced (Figure 2), and binds to the 40S ribosomal subunit. It leads to the inhibition of host translation, and RNA degradation.

In a second aspect of the present invention, the above object is solved by a providing a screening system for identifying a pharmaceutically active compound that modulates the function of a frame-shift pseudoknot element and/or modulates the function of a translation initiation-RNA element of an RNA virus in a mammalian cell, comprising, a mammalian cell comprising a viral RNA comprising a viral frame-shift pseudoknot element and/or a viral translation initiation-RNA element, optionally at least one reporter construct to detect the translation of at least one viral protein, and further optionally, one or more candidate compounds to be tested. The cells may comprise an expression construct for recombinantly expressing at least one RNA structure to be tested, see, for example, below.

In a third aspect of the present invention, the above object is solved by a providing a compound that modulates the function of a frame-shift pseudoknot element and/or modulates the function of a translation initiation-RNA element of an RNA virus in a mammalian cell, identified according to a method according to the present invention, in particular a compound that modulates the function of a frame-shift pseudoknot element of an RNA virus in a mammalian cell according to the following Formula 1 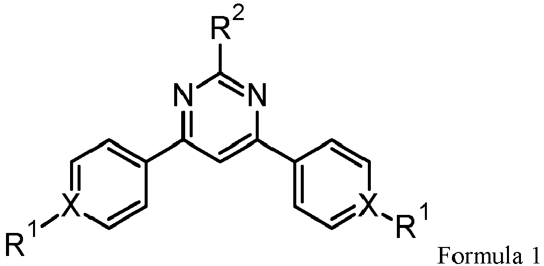
wherein X is independently selected from C or N, R¹ is independently selected from H, -OH, branched or straight C₁ to C₆ alkyl, branched or straight C₁ to C₆ alkyloxy, and -COOH and combinations thereof, R² is selected from 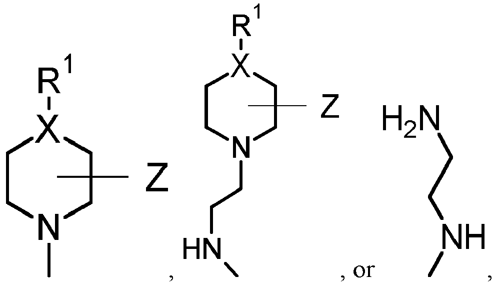
wherein X is as above, R¹ is as above, and Z indicates a mono- or disubstitution with -OH and/or -NH₂, and pharmaceutically acceptable salts and solvates thereof, or a compound that modulates the function of a frame-shift pseudoknot element of an RNA virus in a mammalian cell according to the following Formula 2 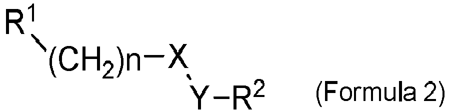
wherein n is an integer from 0 to 3, X and Y are independently selected from -NH-, -C=O- or -CH₂- and R¹ is selected from 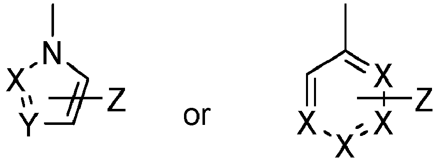
wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
and wherein R² may be attached via C(5), C(6), C(7), or C(8) of the ring structure and is selected from 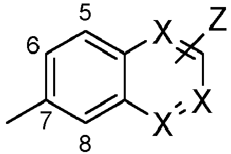
wherein X is independently selected from C or N and Z is as above, and pharmaceutically acceptable salts and solvates thereof, or a compound that modulates the function of a translation-initiation RNA element of an RNA virus in a mammalian cell according to the following Formula 3 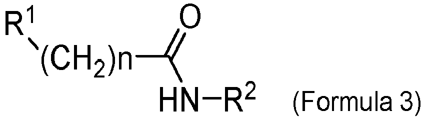
wherein n is an integer from 1 to 3, R¹ is selected from 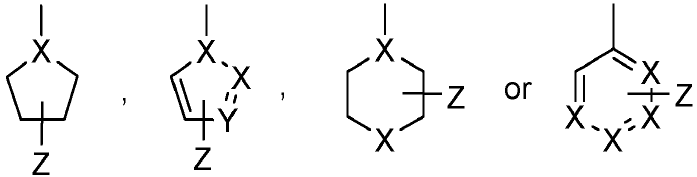
wherein X is selected from C or N, Y is selected from C, N or S and Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
and wherein R² is selected from 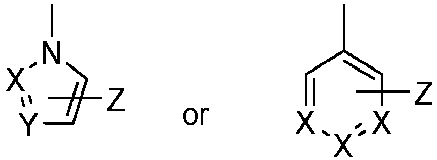
wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof, and pharmaceutically acceptable salts and solvates thereof, or a compound that modulates the function of a translation-initiation RNA element of an RNA virus in a mammalian cell according to the following Formula 4 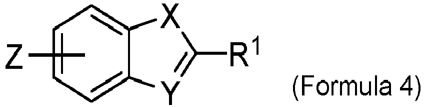
wherein X and Y are selected from C, N or S, Z indicates one or multiple substitution(s) with - H, -OH, branched or straight C₁ to C₆ acyl, branched or straight C₁ to C₆ alkyl, branched or straight C₁ to C₆ alkyloxy and/or -COOH and combinations thereof, and wherein R¹ is selected from 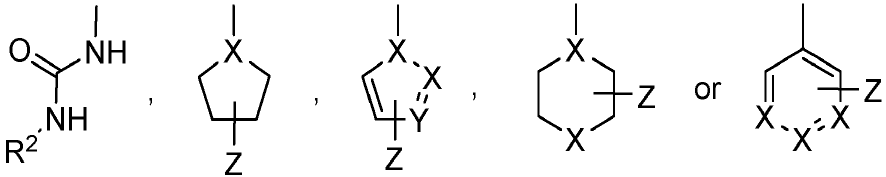
wherein R² is branched or straight C₁ to C₆ alkyl, X is selected from C or N, Y is selected from C, N or S and Z is as above, and pharmaceutically acceptable salts and solvates thereof.

In a fourth aspect of the present invention, the above object is solved by providing a pharmaceutical composition comprising a compound according to the present invention, together with a pharmaceutically acceptable auxiliary agent or carrier.

In a fifth aspect of the present invention, the above object is solved by providing a compound according to the present invention or the pharmaceutical composition according to the present invention for use in the prevention or treatment of a viral infection caused by an RNA virus in a mammalian cell, wherein the mRNA of said RNA virus comprises at least one of a frame-shift pseudoknot element and/or a translation initiation-RNA element. Preferably, the viral infection is selected from an infection with a virus selected from the group consisting of pathogenic RNA viruses, coronaviruses, betacoronaviruses, SARS, MERS, SARS-CoV-2, NL63, OC43, and viral variants and mutants thereof.

In a sixth aspect of the present invention, the above object is solved by a method of modulating the translation of at least one protein of an RNA virus in a mammalian cell, comprising contacting said cell with an effective amount of a compound according to the present invention or a pharmaceutical according to the present invention.

In a seventh aspect of the present invention, the above object is solved by a method of preventing, treating or ameliorating a disease or condition caused by a viral infection caused by an RNA virus in a mammalian cell, wherein the mRNA of said RNA virus comprises at least one of a frame-shift pseudoknot element and/or a translation initiation-RNA element, comprising providing an effective amount of at least one compound according to the present invention or a pharmaceutical according to the present invention to a patient or subject in need of said treatment, amelioration or prevention.

In a first aspect of the present invention, a method for identifying a pharmaceutically active compound that modulates the function of a frame-shift pseudoknot element and/or modulates the function of a translation initiation-RNA element of an RNA virus in a mammalian cell is provided. The method comprises the steps of a) contacting the frame-shift pseudoknot element and/or the translation initiation-RNA element of said RNA virus or a functional fragment thereof with at least one candidate compound, and b) detecting a binding of the at least one candidate compound to the frame-shift pseudoknot element and/or the translation initiation-RNA element of said RNA virus or a functional fragment thereof, wherein a binding of said candidate compound is indicative for a pharmaceutically active compound.

The term "contacting" in the present invention means any interaction between the potential modulator with the RNA element as described herein and/or a recombinant cell expressing said RNA element, whereby any of the two components can be independently of each other in a liquid phase, for example in solution, or in suspension or can be bound to a solid phase, for example, in the form of an essentially planar surface or in the form of particles, pearls or the like. In a preferred embodiment, a multitude of different potentially binding candidate compounds are present in solution and compete for binding to the RNA target.

The method according to the present invention seeks to identify a pharmaceutically active compound that modulates the function of a frame-shift pseudoknot element and/or modulates the function of a translation initiation-RNA element of an RNA virus in a mammalian cell ("modulator"), preferably in a human cell. Conveniently, the format of the method can be quite flexible, the method requires a suitable combination of the two or three components, optionally, a viral protein (either isolated or in combination with other viral components) or a functional fragment thereof, the at least one RNA element to be targeted, and the at least one pharmaceutically active candidate compound, i.e. the substance that shall be screened/identified for the activity to modulate the function of a frame-shift pseudoknot element and/or modulates the function of a translation initiation-RNA element of an RNA virus in a mammalian cell. In the three-component system, the frame-shift pseudoknot element and/or the translation initiation-RNA element thus furthermore includes at least one viral RNA-viral protein complex. The combination can be provided as a cellular system (i.e. functioning in a cell, like in mammalian cell culture), and the components can be provided recombinantly in part or fully, or the system can be in vitro, for example as an in vitro translation system that can be readily adjusted for the purposes of the present invention, if required. Examples are described below.

Preferred is the method according to the present invention, which includes the pre-identification of at least one conserved pseudoknot element and/or translation initiation-RNA element in the viral RNA before the step of contacting with the at least one candidate compound.

The frame-shift pseudoknot element and/or the translation initiation-RNA element of said RNA virus or a functional fragment thereof, as described herein, is then contacted (see above) with said at least one candidate compound, and optionally in the presence of at least one viral protein. Then, the binding of the at least one candidate compound to the frame-shift pseudoknot element and/or the translation initiation-RNA element of said RNA virus or a functional fragment thereof is detected, wherein a binding of said candidate compound is indicative for a pharmaceutically active compound.

It is assumed in the context of the present invention, that binding of said at least one candidate compound to the RNA element or the fragment thereof constitutes an essential step for the modulation function of said compound.

Further, a modulation of the translation of one or more viral proteins of said RNA virus compared to the translation in the absence of said at least one candidate compound can be detected, wherein a modulation of the translation of the said at least one or more viral protein is indicative for a pharmaceutically active compound.

Translation can be detected directly, for example by detecting the amount and/or presence and/or size (length) of the polypeptide as produced. This can be achieved with mass spectrometry, NMR, ELISA, labels that are include into the polypeptide, like labelled amino acids, luminescence constructs (mCherry), fusions (GFP), and the like. Preferably, the detection involves a quantification of the translation product. Specific examples of modulation would be an increase of the translation of a polypeptide as produced by, or the amount or ratio (to each other) of polypeptides translated from a mRNA.

Preferred is the method according to the present invention, wherein the translation of the one or more viral proteins of said RNA virus is essential for viral infection and/or replication. The protein may be selected from an ORF1a, ORF1b or late genes expression product of the genome, such as Nsp1 or envelope proteins.

Preferred is the method according to the present invention, wherein the modulation leads to an increase or decrease of said translation of said at least one viral protein. Preferred is a decrease of the translation, caused by an inhibitor of the viral RNA element(s), which ultimately leads to a decrease of infection.

Further preferred is the method according to the present invention, wherein the RNA virus is selected from the group consisting of pathogenic RNA viruses, coronaviruses, betacoronavirus, SARS, MERS, SARS-CoV-2, NL63, OC43, BCoV, HEV, MHV, HCoV-4408, HCoV-OC43, HCoV-HKU1, and viral variants and mutants thereof as well as BatCoV-HKU2 and other alpha-coronaviruses.

Even further preferred is the method according to the present invention, wherein the pseudoknot element is selected from PK, and the translation initiation-RNA element is selected from 5_SL1, 5_SL2+3, 5_SL4, 5_SL5stem, 5_SL5a, 5_SL5b+c, 5_SL5junc, 5_SL6, 5_SL7, 5_SL8, 5_SL8loop, as well as RNA elements involved in replication 3_SL1, 3_SL2, 3_SL3base, 3_s2m of coronavirus, and viral variants and mutants thereof. Preferred is a translation initiation-RNA element that is selected from 5_SL1, 5_SL4, 5_SL5b+c, 5_SL7, and 5_SL8, more preferably 5_SL1.

Still further preferred is the method according to the present invention, further comprising the step of pre-identifying at least one conserved pseudoknot element and/or translation initiation-RNA element in the viral RNA before the step of contacting with the at least one candidate compound. 3D structures of conserved RNA elements alone and in complex with inhibitors identified may be determined. The methods as described herein can rapidly scan the dynamic conformations of RNAs, which is of crucial relevance for ligand binding thermodynamics and kinetics. For functional validation of the inhibitors targeting RNPs, a particular focus is put on proteins that target unique RNA sequence and structure patterns, as those are engaged in highly viral-specific RNPs and represent bona fide targets for inhibitory compounds. Structure determinations of RNAs and RNPs alone and together with ligands can be carried out by NMR, small angle X-ray scattering (SAXS) or by X-ray crystallography. The majority of coronaviral proteins shows a high degree of sequence and structure conservation, which allows a rapid transfer of the results to related viruses.

Preferred is a method according to the present invention, wherein said contacting with the at least one candidate compound is performed in vitro, in cell culture or in vivo, preferably in a non-human mammal, or comprises in silico molecular modeling, for example using a suitable computer program. Further preferred is a method according to the present invention, furthermore comprising a pre-selection step comprising molecular modeling of said binding of said at least one candidate compound to the pseudoknot element and/or translation initiation-RNA element in the viral RNA or a functional fragment thereof, for example using a computer program, such as SwissDock. That is, the present invention here includes a pre-selection based on the binding properties as modelled in silico, e.g. based on the whole or a part of the structural element, either isolated or in the context of viral proteins, before including compounds in the more complex full in vivo and/or vitro assays.

Physically, the nucleic acid representing the frame-shift pseudoknot element or the translation initiation-RNA element is produced through chemical synthesis or in vitro through enzymatic methods, such as T7 polymerase-based *in-vitro* transcription.

In general, the binding of the candidate compound can be detected using any suitable method known to the person of skill. Preferred is a method according to the present invention, wherein the binding is detected using ligand observed fluorescence titrations (see, for example, Dunn S. (2007) Fluorescence Measurements of Receptor-Ligand Interactions. In: Lajtha A., Baker G., Dunn S., Holt A. (eds) Handbook of Neurochemistry and Molecular Neurobiology. Springer, Boston, MA. https://doi.org/10.1007/978-0-387-30401-4_5).

Methods according to the present invention can be implemented using automation (robotics), and may be performed in a high-throughput format.

The candidate compound and/or that is to be identified or screened in the context of the present invention, can be any chemical substance or any mixture thereof. Preferably, said compound is selected from a chemical substance, a substance selected from a peptide library, a library of small organic molecules, a combinatory library, a cell extract, in particular a plant cell extract, a "small molecular drug" (i.e. having a molecular weight of less than about 500 Da), a repurposable drug, a protein and/or a protein fragment, and an antibody or fragment thereof, and suitable derivatives thereof. Small molecular drugs are preferred.

In the context of the present invention, unless indicated otherwise, the term "about" shall mean +/- 10% of the value as given.

The selected or screened compound can then be modified. Said modification can take place in an additional preferred step of the methods of the invention as described herein, wherein, for example, after analyzing the binding to the RNA elements and/or modulation of the translation, e.g. in the presence and absence of said compound as selected, said compound is further chemically modified as described for example, in the examples below, and analyzed again for its effect on the binding and/or modulation. Said "round of modification(s)" can be performed for one or several times in all the methods, in order to optimize the effect of the compound, for example, in order to improve its specificity for the RNA element to be bound, and/or in order to improve its specificity for the viral translation to be influenced. This strategy is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity, in particular the translational activity of the at least viral protein.

The modification can also be simulated in silico before additional tests are performed in order to confirm or validate the effect of the modified selected or screened compound from the first round of screening. Respective software programs are known in the art and readily available for the person of skill.

Modification can further be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, *n-*propyl, *iso*-propyl, *n*-butyl, *iso*-butyl, *tert*-butyl, *n*-pentyl or *iso*-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group. Again, see examples below for functionally driven modifications.

Therefore, preferred is the method according to the present invention, further comprising c) chemically modifying the candidate compound as identified (see above), and repeating the method according to the present invention in order to identify a second generation candidate compound with improved binding and/or modulating properties, and optionally d) repeating step c) at least once, twice, three times or more.

The inventors further identified that the use of candidate compounds in combination even further improves, and in particular synergistically improves, the effect compared to either drug delivered alone, as the structural RNA elements include several "hot spots" of binding, thus allowing more than one molecule to effectively bind the structure.

The term "functional fragment" shall mean stretches and/or regions of the viral RNA structural elements that are ultimately involved in the modulation of the translation of the at least one viral protein. These areas are involved in binding of the compound (modulator) and/or a subsequent change of the viral protein, e.g. by steric hindrance of the mRNA translation to be produced by the ribosome. Preferred are functional fragments that include the stem-structures as disclosed herein that are useful for binding studies, either for the compound to be tested and/or for pre-screening of the modulator.

In a second aspect of the present invention, the above object is solved by a providing a screening system for identifying a pharmaceutically active compound that modulates the function of a frame-shift pseudoknot element and/or modulates the function of a translation initiation-RNA element of an RNA virus in a mammalian cell, comprising, a mammalian cell comprising a viral RNA comprising a viral frame-shift pseudoknot element and/or a viral translation initiation-RNA element, optionally at least one reporter construct to detect the translation of at least one viral protein, and further optionally, one or more candidate compounds to be tested. The cells may comprise an expression construct for recombinantly expressing at least one RNA structure to be tested, see, for example, below.

Thus, preferred is a screening system according to the present invention that further comprises a recombinant expression construct, preferably an expression vector, for expressing the RNA elements or a fragment thereof, and/or the (in this case proteinaceous or nucleic acid) at least one pharmaceutically active compound to be identified (screened).

Also preferred is a screening system according to the present invention wherein said expression construct for recombinantly expressing viral proteins to be tested further comprises at least one suitable reporter group, such as, for example, luciferase reporters. Most preferred is a dual luciferase reporter system, e.g. of luciferases from *Photinus pyralis* (firefly) and *Renilla reniformis.*

It is preferred that the mammalian cells of the present invention might be selected from a large selection of different human model systems, as long as these are suitable for the analyses as herein.

In support of the methods according to the invention, small molecule binders and prospective modulators of viral translation have been identified. In a third aspect of the present invention, the above object is thus solved by a providing a compound that modulates the function of a frame-shift pseudoknot element and/or modulates the function of a translation initiation-RNA element of an RNA virus in a mammalian cell, identified according to a method according to the present invention, in particular a compound that modulates the function of a frame-shift pseudoknot element of an RNA virus in a mammalian cell according to the following Formula 1 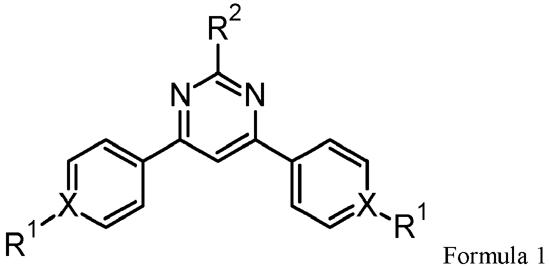
wherein X is selected from C or N, R¹ is selected from H, -OH, branched or straight C₁ to C₆ alkyl, branched or straight C₁ to C₆ alkyloxy, and -COOH, R² is selected from 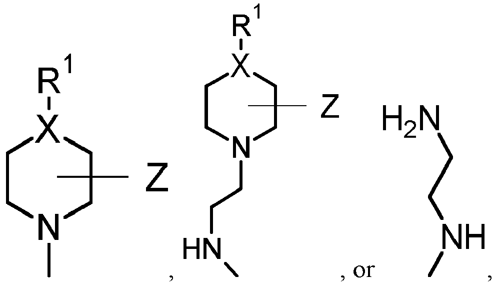
wherein X is as above, R¹ is as above, and Z indicates a mono- or disubstitution with -OH and/or -NH₂, and pharmaceutically acceptable salts and solvates thereof, or a compound that modulates the function of a frame-shift pseudoknot element of an RNA virus in a mammalian cell according to the following Formula 2 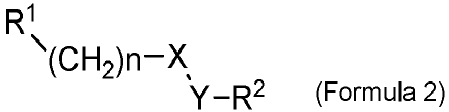
wherein n is an integer from 0 to 3, X and Y are independently selected from -NH-, -C=O- or -CH₂- and R¹ is selected from 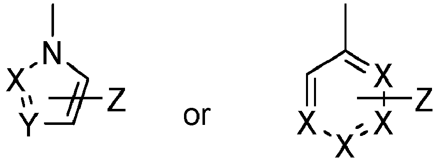
wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
and wherein R² may be attached via C(5), C(6), C(7), or C(8) of the ring structure and is selected from 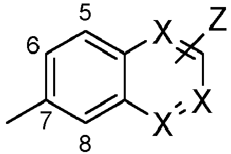
wherein X is independently selected from C or N and Z is as above, and pharmaceutically acceptable salts and solvates thereof, or a compound that modulates the function of a translation-initiation RNA element of an RNA virus in a mammalian cell according to the following Formula 3 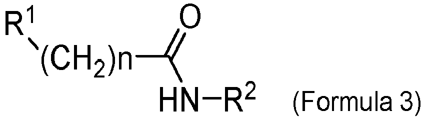
wherein n is an integer from 1 to 3, R¹ is selected from 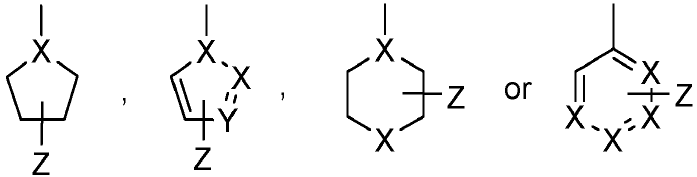
wherein X is selected from C or N, Y is selected from C, N or S and Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
and wherein R² is selected from 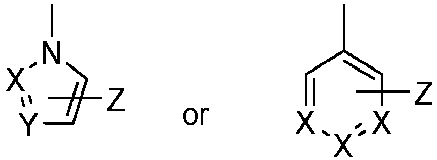
wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof, and pharmaceutically acceptable salts and solvates thereof, or a compound that modulates the function of a translation-initiation RNA element of an RNA virus in a mammalian cell according to the following Formula 4 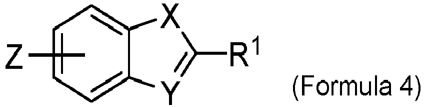
wherein X and Y are selected from C, N or S, Z indicates one or multiple substitution(s) with - H, -OH, branched or straight C₁ to C₆ acyl, branched or straight C₁ to C₆ alkyl, branched or straight C₁ to C₆ alkyloxy and/or -COOH and combinations thereof, and wherein R¹ is selected from 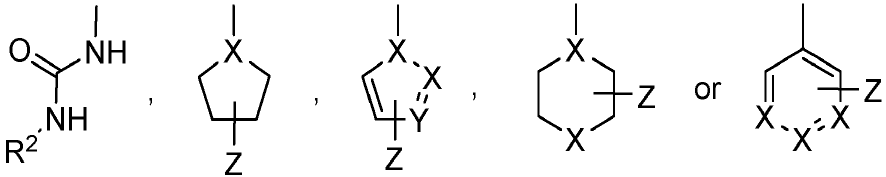
wherein R² is branched or straight C₁ to C₆ alkyl, X is selected from C or N, Y is selected from C, N or S and Z is as above, and pharmaceutically acceptable salts and solvates thereof.

The compound according to the present invention can then be modified. Said modification can take place in an additional preferred step of the methods of the invention as described herein, wherein, for example, after analyzing the binding to the RNA elements and/or modulation of the translation, e.g. in the presence and absence of said compound as selected, said compound is further chemically modified as described for example, in the examples below, and analyzed again for its effect on the binding and/or modulation. Said "round of modification(s)" can be performed for one or several times in all the methods, in order to optimize the effect of the compound, for example, in order to improve its specificity for the RNA element to be bound, and/or in order to improve its specificity for the viral translation to be influenced. This strategy is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity, in particular the translational activity of the at least viral protein.

In the context of the present invention, the term "modulates the function" shall include any change of the element leading to a change of the function based on the change of the structure and/or binding of molecules, such as, for example, a stabilization, inhibition, or activation.

The modification can also be simulated in silico before additional tests are performed in order to confirm or validate the effect of the modified selected or screened compound from the first round of screening. Respective software programs are known in the art and readily available for the person of skill.

Modification can further be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, *n-*propyl, *iso*-propyl, *n*-butyl, *iso*-butyl, *tert*-butyl, *n*-pentyl or *iso*-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group. Again, see examples below for functionally driven modifications.

In a fourth aspect of the present invention, the above object is solved by providing a pharmaceutical composition comprising a compound according to the present invention, together with a pharmaceutically acceptable auxiliary agent or carrier.

Yet another important aspect of the present invention then relates to a method for manufacturing a pharmaceutical composition for the amelioration, prevention or treatment of diseases or condition caused by an RNA virus, comprising the steps of formulating the compound according to the present invention into a suitable pharmaceutical composition, or performing a method according to the present invention for identifying a compound that modulates the function of a frame-shift pseudoknot element and/or modulates the function of a translation initiation-RNA element of an RNA virus in a mammalian cell, and formulating said compound as identified into a suitable pharmaceutical composition. The invention also relates to a pharmaceutical composition obtained by said method according to the present invention. Preferably, said disease or condition to be treated is selected from viral infections caused by an RNA, such as coronavirus, for example SARS-CoV-2.

Thus, in yet another aspect of the present invention, the selected or screened compound and/or compound according to the present invention can be provided and/or is administered as a suitable pharmaceutical composition, such as a tablet, capsule, granule, powder, sachet, reconstitutable powder, dry powder inhaler and/or chewable. Such solid formulations may comprise excipients and other ingredients in suitable amounts. Such solid formulations may contain e.g. cellulose, cellulose microcrystalline, polyvidone, in particular FB polyvidone, magnesium stearate and the like. The modulating compound identified as outlined above, which may or may not have gone through additional rounds of modification, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routes of application like, for example, intravenous solution, sprays, liposomes, ointments, skin creme, band-aids or pills.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound as selected or screened and/or compound for use, if required, as long as they act in combination (i.e. directly and/or indirectly, preferably synergistically) with the present compound as selected and/or screened.

Thus, the compounds according to the present invention or as selected or screened can be used alone or in combination with other active compounds - for example with medicaments already known for the treatment of the aforementioned diseases, whereby in the latter case a favorable additive, amplifying or preferably synergistically effect is noticed. Suitable amounts to be administered to humans range from 5 to 500 mg, in particular 10 mg to 100 mg. Of course, any dosage can be readily adjusted by the attending physician, if needed, based on, for example, other medical parameters of the patient to be treated.

Pharmaceutical compositions as used may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal Si0₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for respective compounds, for example topical, and will readily be able to choose suitable pharmaceutically acceptable carriers or excipients, depending, e.g., on the formulation and administration route of the pharmaceutical composition.

The therapeutics can be administered orally, e.g. in the form of pills, tablets, coated tablets, sugar coated tablets, hard and soft gelatin capsules, solutions, syrups, emulsions or suspensions or as aerosol mixtures. Administration, however, can also be carried out rectally, e.g. in the form of suppositories, or parenterally, e.g. in the form of injections or infusions, or percutaneously, e.g. in the form of ointments, creams or tinctures.

In addition to the aforementioned compounds according to the present invention or as selected or screened, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds of the invention and also other therapeutically active substances as described above.

In another aspect of the present invention, the above object is solved by a method of modulating the translation of at least one protein of an RNA virus in a mammalian cell, comprising contacting said cell with an effective amount of a compound according to the present invention or a pharmaceutical according to the present invention. The modulation can be in vivo or in vitro, and is non-medical, e.g. in the context of a cosmetic use, or for research purposes. Particularly preferred is a method based on a compound that was identified with the enclosed screening systems and/or methods for screening. It is also preferred that the compound according to the present invention can be modified, for example chemically as described herein.

In a fifth aspect of the present invention, the above object is solved by providing a compound according to the present invention or the pharmaceutical composition according to the present invention for use in the prevention or treatment of a viral infection caused by an RNA virus in a mammalian cell, wherein the mRNA of said RNA virus comprises at least one of a frame-shift pseudoknot element and/or a translation initiation-RNA element. Preferably, the viral infection is selected from an infection with a virus selected from the group consisting of pathogenic RNA viruses, coronaviruses, betacoronavirus, SARS, MERS, SARS-CoV-2, HCoV-NL63, BCoV, MHV, HCoV-4408, HCoV-OC43, HCoV-HKU1, and viral variants and mutants thereof.

In another important aspect of the present invention, the above object is solved by a method of preventing, treating or ameliorating a disease or condition caused by a viral infection caused by an RNA virus in a mammalian cell, wherein the mRNA of said RNA virus comprises at least one of a frame-shift pseudoknot element and/or a translation initiation-RNA element, comprising providing an effective amount of at least one compound according to the present invention or a pharmaceutical according to the present invention to a patient or subject in need of said treatment, amelioration or prevention.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

As above, the compound as administered in the context of the present invention can be any chemical substance or any mixture thereof. Preferably, said compound is selected from a substance selected from a peptide library, a library of small organic molecules (i.e. of a molecular weight of about 500 Da or less), a combinatory library, a cell extract, in particular a plant cell extract, a small molecular drug, a protein and/or a protein fragment, and an antibody or fragment thereof, and in particular from a compound according to the present invention as above and derivatives thereof.

Preferred is a method according to the present invention, wherein said disease or condition is a viral infection with a virus selected from the group consisting of pathogenic RNA viruses, coronaviruses, betacoronavirus, SARS, MERS, SARS-CoV-2, HCoV-NL63, BCoV, MHV, HCoV-4408, HCoV-OC43, HCoV-HKU1, and viral variants and mutants thereof, in particular SARS-CoV -2.

Small molecule therapeutics targeting defined RNA structural elements are of high potential. Not only when targeting RNA viruses, but in the treatment of human diseases in general, as aberrant gene expression changes, which can be effectively controlled at RNA level, are at the core of pathological disease states. Only recently, substantial progress has been reported in the development of RNA-targeting drugs correcting defective splicing in diseases like spinal muscular atrophy (SMA) and Huntington's disease (HD). First approvals by the FDA have been granted in 2020 to the drugs Risdiplam (Genentech/Roche) and Branaplam (Novartis). This highlights the enormous potential of RNA-targeting drugs, which the inventors are just beginning to harvest.

Even for the vast numbers of emerging viruses with RNA genomes, the RNA target space can be determined rapidly once the sequence becomes available. Then, this target space and its changes in occurring variants of concern (VOCs) can be monitored quickly, e.g. within two weeks. From such analyses, it is the next step to focus on the parts of the RNA genome that display enhanced evolutionary conservation compared to the overall mutation rate of the viral genome. As these frequently noncoding RNAs are conserved during the evolution of viruses and are important for the viral lifecycle, their inhibition becomes a deadly weapon against viruses.

Within the non-coding genomic regions, a substantial number of RNA regulatory motifs can be identified. This function is linked to intricate 3D folded structures providing specific recognition sites for antiviral drugs. Next to several approved therapeutics exploiting an antisense strategy, which targets RNA in a sequence-specific manner, molecules of low molecular weight (small molecules, drugs) are attractive candidates to specifically target RNA motifs with non-canonical structure. In fact, cell-based assays have shown that small molecule interference with the biological functions of these viral RNA regulatory elements is detrimental to virus propagation and pathogenicity.

Coronaviruses contain a large, positive single-stranded RNA genome with a length of ~ 30 kb. For SARS-CoV-2, the architecture of the RNA genome has been rapidly and intensively investigated by chemical probing approaches, both in vitro and in vivo. These approaches demonstrated the presence of secondary structure elements throughout the genomic RNA of SARS-CoV-2 and in particular in its 5'- and 3'-untranslated regions (UTRs). These RNA structures as well as the frameshifting region between the overlapping reading frames 1a and 1b are essential for genome replication, regulation of viral translation and hijacking of the host-cell biomolecular machinery to promote virus propagation. The functional RNA elements are conserved between human transmissible coronavirus strains and thus represent an underexploited class of high potential drug targets to tackle not only SARS-CoV-2, but also existing (e.g. MERS) as well as future pathogenic coronavirus strains.

RNA structure probing tools used to study the SARS-CoV-2 RNA are well suited to identify stable base-paired helical elements. Thus, they allow the quick identification of changes in base-pairing patterns as a result of genomic mutations in uprising VOCs. Structural probing analysis revealed (i) that the structural elements in the genomic RNA are organized in a modular fashion with local structured elements connected by single-stranded regions, (ii) that they are conserved between related coronaviruses and thus their structure is of biological significance, and (iii) that structural probing results are widely consistent between in vitro transcribed RNA elements and full-length genomic RNA present in vivo in infected cells facilitating the use of elements in in vitro high-throughput drug discovery campaigns.

For structure-guided design of drugs interfering with RNA function, however, additional information on the 3D structure and dynamics of the targeted RNA is crucial. For that, NMR-spectroscopy in solution is the method of choice. This is because RNAs adopt multiple structures in cellular environments as well as in solution, in particular in non-canonical structural elements. The analysis of individual RNA targets can be extended to detailed structure determination of RNA-protein complexes (RNPs). Structures of either RNAs or RNPs will drive the medicinal chemistry. The present approach develops RNA-drugs that target non-canonical dynamic structures of an RNA virus and characterize suitable pockets for RNA-drugs.

The present invention preferably relates to the following items:
Item 1. A method for identifying a pharmaceutically active compound that modulates the function of a frame-shift pseudoknot element and/or modulates the function of a translation initiation-RNA element of an RNA virus in a mammalian cell, comprising
   a) contacting the frame-shift pseudoknot element and/or the translation initiation-RNA element of said RNA virus or a functional fragment thereof with at least one candidate compound, and
   b) detecting a binding of the at least one candidate compound to the frame-shift pseudoknot element and/or the translation initiation-RNA element of said RNA virus or a functional fragment thereof,
   wherein a binding of said candidate compound is indicative for a pharmaceutically active compound.
Item 2. The method according to Item 1, wherein the frame-shift pseudoknot element and/or the translation initiation-RNA element furthermore includes at least one viral RNA-viral protein complex.
Item 3. The method according to Item 1 or 2, further comprising the step of detection a modulation of the translation of one or more viral proteins of said RNA virus compared to the translation in the absence of said at least one candidate compound, wherein a modulation of the translation of the said at least one or more viral protein is indicative for a pharmaceutically active compound.
Item 4. The method according to Item 3, wherein the translation of the one or more viral proteins of said RNA virus is essential for viral infection and/or replication.
Item 5. The method according to Item 3 or 4, wherein the modulation leads to an increase or decrease of said translation of said at least one viral protein.
Item 6. The method according to any one of Items 1 to 5, wherein the RNA virus is selected from the group consisting of pathogenic RNA viruses, coronaviruses, betacoronaviruses, SARS, MERS, SARS-CoV-2, HCoV-NL63, BCoV, MHV, HCoV-4408, HCoV-OC43, HCoV-HKU1, and viral variants and mutants thereof.
Item 7. The method according to any one of Items 1 to 6, wherein the pseudoknot element is selected from PK, and the translation initiation-RNA element is selected from 5_SL1, 5_SL2+3, 5_SL4, 5_SL5stem, 5_SL5a, 5_SL5b+c, 5_SL5junc. 5_SL6, 5_SL7, 5_SL8, 5_SL8loop, 3_SL1, 3_SL2, 3_SL3base, 3_s2m of coronavirus, and viral variants and mutants thereof.
Item 8. The method according to any one of Items 1 to 7, further comprising the step of pre-identifying at least one conserved pseudoknot element and/or translation initiation-RNA element in the viral RNA before the step of contacting with the at least one candidate compound.
Item 9. The method according to any one of Items 1 to 8, wherein said contacting with the at least one candidate compound is performed in vitro, in cell culture or in vivo, preferably in a non-human mammal, or comprises in silico molecular modeling, for example using a suitable computer program.
Item 10. The method according to any one of Items 1 to 9, wherein the frame-shift pseudoknot element or the translation initiation-RNA element is produced by T7 polymerase-based *in-vitro* transcription or RNA synthesis methods.
Item 11. The method according to any one of Items 1 to 10, wherein the binding is detected using ligand observed fluorescence titrations.
Item 12. The method according to any one of Items 1 to 11, wherein said candidate compound is selected from a chemical substance, a substance selected from a peptide library, a library of small organic molecules, a combinatory library, a cell extract, in particular a plant cell extract, a "small molecular drug", a repurposable drug, a protein and/or a protein fragment, and an antibody or fragment thereof, and suitable derivatives thereof.
Item 13. The method according to any one of Items 1 to 12, further comprising c) chemically modifying the candidate compound as identified, and repeating the method according to any one of Items 1 to 12 in order to identify a second generation candidate compound with improved modulating properties, and optionally d) repeating step c) at least once.
Item 14. A screening system for identifying a pharmaceutically active compound that modulates the function of a frame-shift pseudoknot element and/or modulates the function of a translation initiation-RNA element of an RNA virus in a mammalian cell, comprising,
   a mammalian cell comprising a viral RNA comprising a viral frame-shift pseudoknot element and/or a viral translation initiation-RNA element,
   optionally at least one reporter construct to detect the translation of at least one viral protein, and
   further optionally, one or more candidate compounds to be tested.
Item 15. The screening system according to Item 14, wherein said mammalian cell is selected from a hamster or human cell.
Item 16. A compound that modulates the function of a frame-shift pseudoknot element and/or modulates the function of a translation initiation-RNA element of an RNA virus in a mammalian cell, identified according to a method according to any one of Items 1 to 13, in particular a compound that modulates the function of a frame-shift pseudoknot element of an RNA virus in a mammalian cell according to the following Formula 1 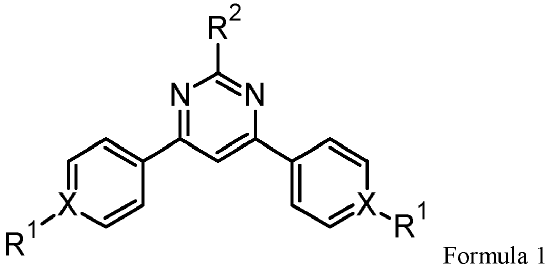
   wherein X is selected from C or N, R¹ is selected from H, -OH, branched or straight C₁ to C₆ alkyl, branched or straight C₁ to C₆ alkyloxy, and -COOH, R² is selected from 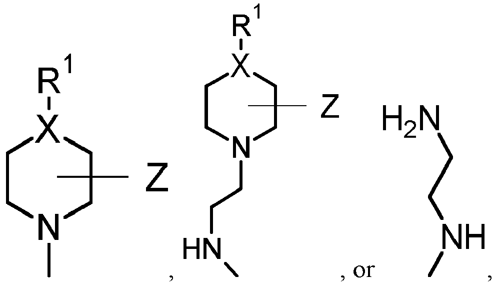
   wherein X is as above, R¹ is as above, and Z indicates a mono- or disubstitution with -OH and/or -NH₂, and pharmaceutically acceptable salts and solvates thereof, or a compound that modulates the function of a frame-shift pseudoknot element of an RNA virus in a mammalian cell according to the following Formula 2 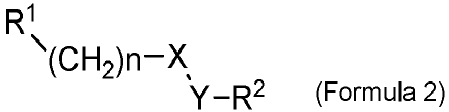
   wherein n is an integer from 0 to 3, X and Y are independently selected from -NH-, -C=O- or -CH₂- and R¹ is selected from 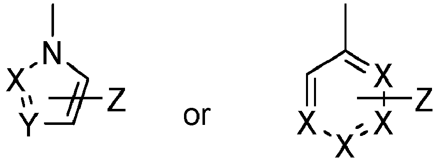
   wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
   and wherein R² may be attached via C(5), C(6), C(7), or C(8) of the ring structure and is selected from 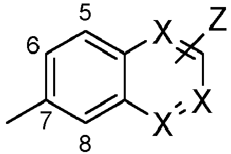
   wherein X is independently selected from C or N and Z is as above, and pharmaceutically acceptable salts and solvates thereof, or a compound that modulates the function of a translation-initiation RNA element of an RNA virus in a mammalian cell according to the following Formula 3 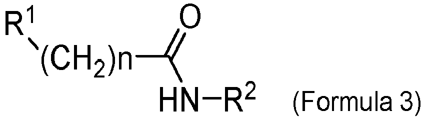
   wherein n is an integer from 1 to 3, R¹ is selected from 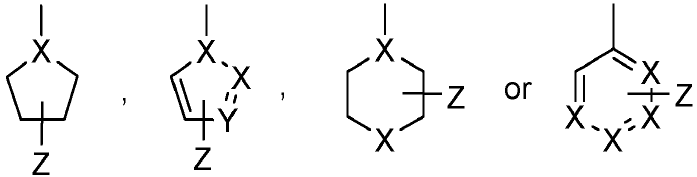
   wherein X is selected from C or N, Y is selected from C, N or S and Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
   and wherein R² is selected from 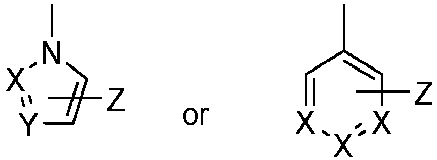
   wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof, and pharmaceutically acceptable salts and solvates thereof, or a compound that modulates the function of a translation-initiation RNA element of an RNA virus in a mammalian cell according to the following Formula 4 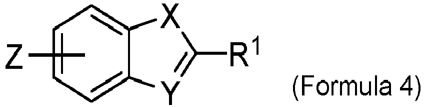
   wherein X and Y are selected from C, N or S, Z indicates one or multiple substitution(s) with - H, -OH, branched or straight C₁ to C₆ acyl, branched or straight C₁ to C₆ alkyl, branched or straight C₁ to C₆ alkyloxy and/or -COOH and combinations thereof, and wherein R¹ is selected from 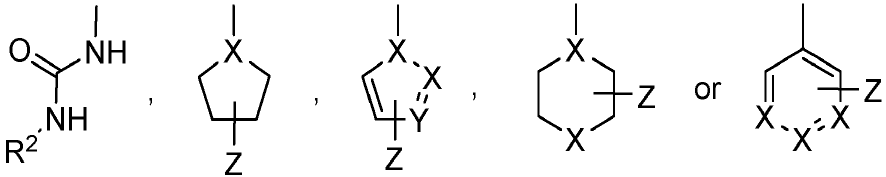
   wherein R² is branched or straight C₁ to C₆ alkyl, X is selected from C or N, Y is selected from C, N or S and Z is as above, and pharmaceutically acceptable salts and solvates thereof.
Item 17. A pharmaceutical composition comprising a compound according to Item 16, together with a pharmaceutically acceptable auxiliary agent or carrier.
Item 18. A compound according to claim 16 or the pharmaceutical composition according to Item 17 for use in the prevention or treatment of a viral infection caused by an mRNA virus in a mammalian cell, wherein the mRNA of said mRNA virus comprises at least one of a frame-shift pseudoknot element and/or a translation initiation-RNA element.
Item 19. The compound or the pharmaceutical composition for use according to Item 18, wherein said compound is selected from a chemical substance, a substance selected from a peptide library, a library of small organic molecules, a combinatory library, a cell extract, in particular a plant cell extract, a small molecular drug, a protein and/or a protein fragment, and an antibody or fragment thereof, and in particular from atazanavir and derivatives thereof and artesunate and derivatives thereof.
Item 20. The compound or the pharmaceutical composition for use according to Item 18 or 19, wherein the RNA virus is selected from the group consisting of pathogenic RNA viruses, coronaviruses, betacoronaviruses, SARS, MERS, SARS-CoV-2, HCoV-NL63, BCoV, MHV, HCoV-4408, HCoV-OC43, HCoV-HKU1, and viral variants and mutants thereof.
Item 21. The compound or the pharmaceutical composition for use according to any one of Items 18 to 20, wherein the pseudoknot element is selected from PK, and the translation initiation-RNA element is selected from 5_SL1, 5_SL2+3, 5_SL4, 5_SL5stem, 5_SL5a, 5_SL5b+c, 5_SL6, 5_SL7, 5_SL8, 5_SL8loop, 3_SL1, 3_SL2, 3_SL3base, 3_s2m of coronavirus, and viral variants and mutants thereof.
Item 22. A method of modulating the translation of at least one protein of an RNA virus in a mammalian cell, comprising contacting said cell with an effective amount of a compound according to Item 16 or a pharmaceutical according to Item 17.
Item 23. A method of preventing, treating or ameliorating a disease or condition caused by a viral infection caused by an RNA virus in a mammalian cell, wherein the mRNA of said RNA virus comprises at least one of a frame-shift pseudoknot element and/or a translation initiation-RNA element, comprising providing an effective amount of at least one compound according to Item 16 or a pharmaceutical according to Item 17 to a patient or subject in need of said treatment, amelioration or prevention.

The present invention will now be described further in the following examples with reference to the accompanying Figures, nevertheless, without being limited thereto. For the purposes of the present invention, all references as cited herein are incorporated by reference in their entireties.

Figure 1 shows a schematic overview of *cis*-acting RNA elements of the SARS-CoV-2 genome. Black: untranslated regions; orange: coding regions. The SL structures investigated in the context of the invention and their relative positions within the SARS-CoV-2 genome are shown schematically. "N" represents the end of ORF9 coding for the Nucleocapsid protein.

Figure 2 shows a schematic overview over the function of the viral early stage protein NSP1 in hijacking the eukaryotic ribosome for viral replication. G = Guide.

Figure 3 shows the determination of the binding of compound D01 to the pseudoknot structure, as well as the chemical structures of optimized compounds D01+DAP (KD = 300 nM for D01+DAP), and D05 (K_{D}: 140 µM for D05).

Figure 4 shows the determination of the binding of compound D01+DAP to the pseudoknot structure. A: NMR-data for the chemical shift perturbation to map the binding site of ligand D01+DAP;
PK: 5'-GGCCAGUAGGCGGUGUAAGUGCAGCCCGUCUUACACCGUGCGGCAC-AGGCACUAGUACUGAUGUCGUAUACAGGGCU-3' (SEQ ID NO: 1)
B: in-line probing with sequence Cy5-T10-R8-PK: 5'-Cy5-TTTTTTTTTT-GGCCAGUA-GGCGGUGUAAGUGCAGCCCGUCUUACACCGUGCGGCACAGGCACUAGUACUGA UGUCGUAUACAGGGCU-3' (SEQ ID NO: 2)

Figure 5 shows data for the 5_SL1 sequence blocking virus-induced translation shut-down. The sequence is 5'-GGGUUUAUACCUUCCCAGGUAACAAACCC-3' (SEQ ID NO: 3).
A: NMR data for an exemplary compound (C11) binding to 5_SL1. B: determination of the binding of the exemplary compound C11 as shown to the 5_SL1 structure. KD = 110.4 +/- 44.2 µM.

Figure 6 shows data for the fluorescence translation initiation efficiency assay, with or without viral protein nsp1, and with or without inhibitor (C11) as shown.

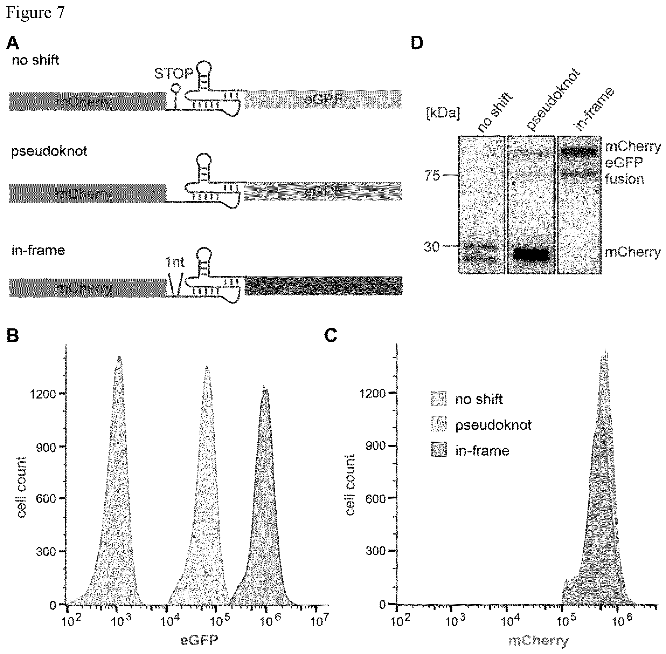

Figure 7 shows the components of a frameshifting assay to be used as an alternative for the pseudoknot analysis. The assay is a FACS-based fluorescence reporter assay, which preferably is used in a 96-well format. It uses genomic integration (site-specific, single copy), and Western blot for protein quantification. A) Scheme of the frameshifting reporter gene constructs: no shift = contains a STOP codon in the slippery sequence, pseudoknot = wild type SARS-CoV-2 pseudoknot + slippery sequence, in-frame = contains a 1 nt insertion in the slippery sequence. B) and C) FACS quantification of eGFP (B) and mCherry (C) fluorescence of reporters shown in (A). All reporters show equal mCherry levels, while eGFP levels are dependent on frameshifting. D) Western blot assay of mCherry and mCherry-eGFP fusion proteins of reporters shown in (A).

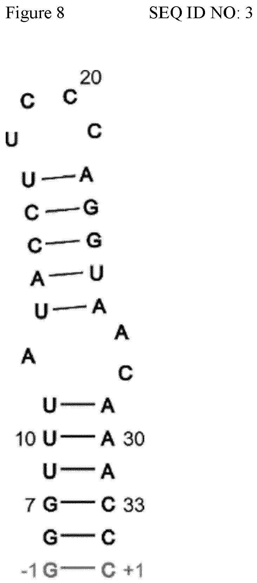

Figure 8 shows the predicted secondary structure of the SARS-CoV-2 5_SL1 5'-terminal structural element, nucleotides 7-33, a *cis*-regulatory element conserved among betacoronaviruses.

Figure 9 shows A) the synthesis scheme for the symmetrical pyrimidine precursors for binders (see examples below), and B) the synthesis scheme for the asymmetrical pyrimidine precursors for binders (see examples below).

Figure 10 shows the synthesis scheme for precursor d (see examples below).

Figure 11 shows A) the synthesis scheme for precursor e (see examples below), and B) the synthesis scheme for precursor k (see examples below).

Figure 12 shows the general synthesis scheme for the coupling of pyrimidine precursors with cyclic and acyclic amines d-g under S_{N}Ar conditions (see examples below).

Figure 13 shows the synthesis scheme for the coupling of pyrimidine precursors with k under *Suzuki-Miyaura* conditions (see examples below).

Figure 14 shows that compound D01+DAP has the highest binding affinity to the SARS-CoV-2 pseudoknot, which was examined via ligand observed fluorescence titrations.

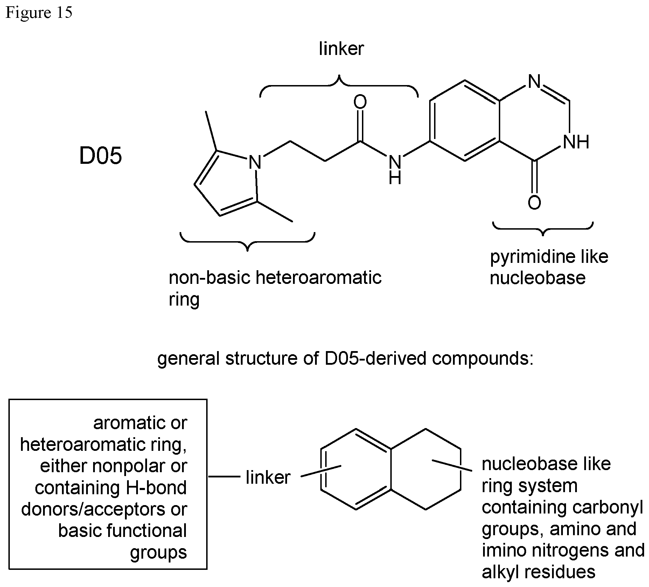

Figure 15 shows an overview over the three parts of molecule D05: a non-basic heteroaromatic ring, a linker and a pyrimidine like base connected via an aromatic ring to the linker.

Figure 16 shows examples of nucleobase-like ring systems suitable for the synthesis of, for example, D05-analog compounds for optimization and testing.

Figure 17 shows A) the results of the performed *in silico* FTMap experiments. Resulting crossclusters were mapped to the three-dimensional structure of the SARS-CoV-2 pseudoknot. Two separate binding hotspots (blue) could be identified. And B) mapping of the experimental results obtained via ¹H,¹⁵N-HSQC NMR experiments (green) and inline probing experiments (purple).

### Examples

### Monitoring and expanding the RNA target space by structure probing

Structural models for many of the RNA elements conserved in RNA viruses, such as SARS-CoV-2, can be provided by *in silico* methods using RNA structure prediction, mutational covariance analysis or homology models according to the methods disclosed in (Rangan R., et al. RNA genome conservation and secondary structure in SARS-CoV-2 and SARS-related viruses: a first look. RNA. 2020; 26: 937-959; Rangan R., et al. De novo 3D models of SARS-CoV-2 RNA elements from consensus experimental secondary structures. Nucleic Acids Research 2021; 49: 3092-3108; Andrews R., et al. An in silico map of the SARS-CoV-2 RNA Structurome. 2020; bioRxiv doi: 18 April 2020, preprint: not peer reviewed https://doi.org/10.1101/2020.04.17.045161).

The secondary structures of all conserved *cis*-acting RNA elements of SARS-CoV-2 are then analyzed by nuclear magnetic resonance (NMR) spectroscopy, providing high-resolution experimental secondary structure models.

The overall folding of viral RNA genomes may also be mapped by chemical footprinting (DMS- and SHAPE-Seq), and changes in RNA fragments of up to 500 nucleotides covering regions of interest are analyzed. This method can be readily adapted to screen for changes in RNA elements in the genome of virtually any RNA virus and quickly probe sequences containing newly identified regulatory RNA motifs.

The identified RNA elements are either novel or affected by mutations that lead to major changes in structure, are characterized by NMR in order to fine map shifts in the 3D folds. These analyses allow to define conserved RNA elements as privileged target sites for the development of antiviral drugs and also flexibly adapts the method to emerging variants.

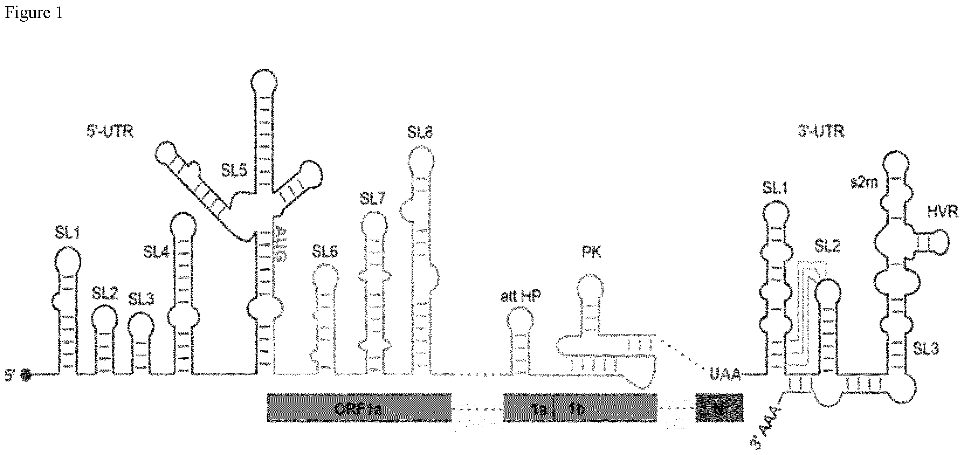

For the methods herein, nine RNA constructs representing the eight stem-loop (SL) domains present at the genomic 5'-end, two RNA constructs corresponding to *cis*-acting elements from the ORF1a/b frameshifting region and four RNA constructs representing functional SLs within the viral 3'-UTR (Figure 1) were identified and analyzed. These 15 RNA elements were chosen based on their conservation within the Betacoronavirus family and their importance for viral propagation.

3D structures of conserved RNA elements alone and in complex with inhibitors identified are determined. The method herein can rapidly scan the dynamic conformations of RNAs, which is of crucial relevance for ligand binding thermodynamics and kinetics. For functional validation of the inhibitors targeting RNPs, a particular focus is put on proteins that target unique RNA sequence and structure patterns, as those are engaged in highly viral-specific RNPs and represent bona fide targets for inhibitory compounds.

Structure determinations of RNAs and RNPs alone and together with ligands is carried out by NMR, small angle X-ray scattering (SAXS) or by X-ray crystallography. The majority of coronaviral proteins shows a high degree of sequence and structure conservation, which allows rapid transfer towards related viruses.

For methods to be used, also see Sreeramulu, Sridhar et al. (in: "Exploring the Druggability of Conserved RNA Regulatory Elements in the SARS-CoV-2 Genome." Angewandte Chemie (International ed. in English) vol. 60,35 (2021): 19191-19200. doi:10.1002/anie.202103693) disclose that in the genome of SARS-CoV-2, 15 RNA elements were identified as conserved between SARS and SARS-CoV-2. By nuclear magnetic resonance (NMR) spectroscopy, they determined that these elements fold independently, in line with data from in vivo and *ex vivo* structural probing experiments. These elements contain non-base-paired regions that potentially harbor ligand-binding pockets. They performed an NMR-based screening of a poised fragment library of 768 compounds for binding to these RNAs, employing three different ¹H-based 1D NMR binding assays. The screening identified common as well as RNA-element specific hits. The results allow selection of the most promising of the 15 RNA elements as putative drug targets. Based on the identified hits, they derived key functional units and groups in ligands for effective targeting of the RNA of SARS-CoV-2.

For methods to be used, also see Richter C, Hohmann KF, Toews S, et al. (in: 1H, 13C and 15N assignment of stem-loop SL1 from the 5'-UTR of SARS-CoV-2. Biomol NMR Assign. 2021;15(2):467-474. doi: 10.1007/s12104-021-10047-2) disclose that the stem-loop (SL1) is the 5'-terminal structural element within the single-stranded SARS-CoV-2 RNA genome. It is formed by nucleotides 7-33 and consists of two short helical segments interrupted by an asymmetric internal loop. This architecture is conserved among Betacoronaviruses. SL1 is present in genomic SARS-CoV-2 RNA as well as in all subgenomic mRNA species produced by the virus during replication, thus representing a ubiquitous *cis*-regulatory RNA with potential functions at all stages of the viral life cycle. They present the ¹H, ¹³C and ¹⁵N chemical shift assignment of the 29 nucleotides-RNA construct 5_SL1, which denotes the native 27mer SL1 stabilized by an additional terminal G-C base-pair.

While the present examples have been performed with the sequence of SARS-CoV-2 RNA, the present methods can be readily adjusted to other RNA viral sequences.

### DMS footprinting

A total of 5 µl of 200 ng/µl purified SARS-CoV-2 5'-end and 3 '-UTR RNA were heated to 95°C for 15 s and flash cooled on ice for 2 min. A total of 95 µl DMS modification buffer (100 mM sodium cacodylate, 140 mM KCl, 3 mM MgCl₂, pH 7.5) was added to the RNA sample and incubated for 30 min at room temperature. Two microliter of DMS was added, and the mixture was incubated at 37°C with 500 rpm shaking for 10 min. The methylation reaction was terminated by adding 60 µl of neat β-mercaptoethanol (Sigma-Aldrich). The modified RNA samples were purified and desalted using the RNA-cleanup-and-concentrator-5 column kit (Zymo Research) to recover RNAs containing more than 200 nts.

Methylated RNAs were used for reverse transcription to generate DNA with thermostable group II intron reverse transcriptase, third generation (TGIRT-III, InGex), following the manufacturer's protocol. Reverse primers for SARS-CoV-2 5'-end and 3'-UTR, respectively, were commercially synthesized (IDT). The RNA templates were digested using RNase H (NEB) for 20 min at 37°C following the reverse transcription. The synthesized DNA was sequentially polymerase chain reaction (PCR) amplified using Phusion DNA polymerase (NEB) with specific primer sets. The following PCR protocol was applied: denaturing for 30 s at 98°C, followed by 30 PCR cycles, including denaturing for 5 s at 98°C, annealing for 10 s at optimal temperature and extension for 15 s at 72°C; final extension continued for 5 min at 72°C. PCR products were desalted using the DNA-cleanup-and-concentrator-5 column kit (Zymo Research). The homogeneity of the amplified samples was verified using agarose gel electrophoresis prior to sending out for sequencing.

Sequencing was performed on an Illumina *HiSeq 2000* system, which used cluster generation and sequencing by synthesis (SBS) chemistry. The sequence results of SARS-CoV-2 5'-genomic region and 3'-UTR RNA were aligned against index files and then used to generate the population average and read coverage quality control files. The mutational signal of 5' and 3' primer overlapping regions and signal from T and G nucleotides in the sequence were determined to be below background. The signals from A and C nucleotides were normalized to the highest reactivity following 95% Winsorization. The DMS restraint files for each RNA were used as input to guide folding of full-length SARS-CoV-2 5'-end and 3'-UTR with the fold algorithm from the RNAStructure (Bellaousov S., et al. RNAstructure: web servers for RNA secondary structure prediction and analysis. Nucleic Acids Res. 2013; 41: W471) webserver and visualized by VARNA (Darty K. Denise A., Ponty Y. VARNA: interactive drawing and editing of the RNA secondary structure. Bioinformatics. 2009; 25:1974-1975).

### RNA synthesis for NMR experiments

Fast RNA production was achieved by distribution and parallelization of individual synthesis steps. In general, all RNAs were produced by T7 polymerase-based *in-vitro* transcription (Milligan J.F., Uhlenbeck O.C. Synthesis of small RNAs using T7 RNA polymerase. Methods Enzymol. 1989; 180:51-62.). In the DNA template production step, the sequences of 5_SL1, 5_SL2+3, 5_SL4, 5_SL5stem, 5_SL5a, 5_SL5b+c, 5_SL6, 5_SL7, 5_SL8, 5_SL8loop, PK, 3_SL1, 3_SL2, 3_SL3base and 3_s2m together with the T7 promoter were generated by hybridization of complementary oligonucleotides and introduced into the EcoRI and Ncol sites of an HDV ribozyme encoding plasmid based on the pSP64 vector (Promega). The DNA template for 5_SL1-4 was produced by PCR amplification from the full-length 5'-genomic region. RNAs were transcribed as HDV ribozyme fusions to obtain 3' homogeneity. The DNA sequences of the full-length 5'-genomic region, the 3'-UTR and the hypervariable region (3_HVR) were purchased from Eurofins Genomics and introduced into the EcoRI and HindIII sites of the pSP64 vector (Promega). The DNA template for att HP together with the T7 promoter was generated by hybridization of complementary oligonucleotides and directly used for run-off transcription. Isolated RNA hairpins 5_SL5b and 5_SL5c were purchased from Dharmacon and purified according to the manufacturer's instructions.

The recombinant vectors were transformed and amplified in *Escherichia coli* strain DH5α. Plasmid-DNA (2-10 mg plasmid per liter SB medium) was purified by Gigaprep (Qiagen) according to the manufacturer's instructions and linearized with *Hind*III (or *Sma*I in case of 3_HVR) prior to *in-vitro* transcription by T7 RNA polymerase (P266L mutant)). Four preparative-scale transcriptions (10-15 ml each) were routinely performed in parallel. The RNAs were purified as follows: preparative transcription reactions (6 h at 37°C) were terminated by addition of ethylenediaminetetraacetic acid (EDTA) and RNAs were precipitated with 2-propanol. RNA fragments were separated on 10-12% denaturing polyacrylamide (PAA) gels and visualized by UV shadowing at 254 nm. Desired RNA fragments were excised from the gel and RNA was eluted by passive diffusion into 0.3 M NaOAc, precipitated with EtOH or EtOH/acetone and desalted via PD10 columns (GE Healthcare). Except for the att HP RNA, residual PAA was removed by reversed-phase HPLC using a Kromasil RP 18 column and a gradient of 0-40% 0.1 M acetonitrile/triethylammonium acetate. After freeze-drying of RNA-containing fractions and cation exchange by LiClO₄ precipitation (2% in acetone), each RNA was folded in water by heating to 80°C followed by rapid cooling on ice except for the pseudoknot RNA, which was kept below 40°C to optimize folding into monomeric form. Buffer exchange to NMR buffer (25 mM potassium phosphate buffer, pH 6.2, 50 mM potassium chloride) was performed using centrifugal concentrators with a suitable molecular weight cutoff membrane. RNA purity was verified by denaturing PAA gel electrophoresis and homogenous folding was monitored by native PAA gel electrophoresis, loading the same RNA concentration as used for NMR experiments.

The RNA constructs representing the entire 5'-genomic end and the entire 3 '-UTR were purified without denaturing steps in order to maintain the native fold. Thus, transcription reactions for 5'-geRNA and 3 '-UTR were terminated through addition of EDTA, and RNA was extracted by aqueous phenolic extraction at pH 4. Phenol was removed by three cycles of chloroform extraction and subsequent buffer exchange via PD10 columns equilibrated with NMR buffer. The RNAs were purified by size exclusion chromatography using a HiLoad 26/60 Superdex 200 pg column. Fractions containing RNAs of the proper size were identified by denaturing and native gel electrophoresis, pooled and concentrated by centrifugal concentrators (MWCO: 50 kDa).

The RNA constructs were purified by 8-10% denaturing polyacrylamide gel electrophoresis depending on the size of the RNA and eluted in Tris-borate-EDTA (TBE) buffer. The RNA samples were desalted and adjusted to <20 µM concentration using Nanodrop (ThermoFisher Scientific). All desalted RNA samples were annealed in RNase-free water by heating the sample to 95°C for 2 min followed by snap cooling on ice for a minimum 15 min. The annealed samples were concentrated using a centrifugation filtration system (Amicon) and exchanged into NMR buffer. For isotope labeled samples, uniformly ¹³C,¹⁵N-labeled uridine (rUTP) and guanosine (rGTP) (Cambridge Isotope Laboratories), and unlabeled adenosine (rATP) and cytidine (rCTP) (Sigma-Aldrich) were used in the preparative transcriptions. Comparison of RNAs prepared in different laboratories yielded close-to-identical NMR spectra, demonstrating the value of the coordinated research approach within the NMR consortium Covid19-NMR.

### NMR experiments

NMR measurements were carried out on Bruker 600, 800, 900 and 950 MHz AVIIIHD or AV NEO spectrometers equipped with cryogenic triple-resonance HCN probes, on a Bruker 700 MHz AVIIIHD spectrometer equipped with a cryogenic quadruple-resonance QCI-P probe and a Bruker 800 MHz AVIII spectrometer equipped with a carbon optimized TXO cryogenic probe. All probes at BMRZ have z-axis pulsed-field gradient accessory. Data were processed and analyzed using TOPSPIN 4.0 (Bruker BioSpin, Germany) or NMRpipe/NMRDraw. NMRFAM-SPARKY, CccpNmr or CARA (http://www.cara.nmr-software.org/downloads/3-85600-112-3.pdf) were used for chemical shift assignment.

The conducted NMR experiments yielded the following information: ¹H,¹H-NOESY experiments correlate signals from RNA protons through space within up to 5.0 Å spatial proximity. In A-form RNA helices, sequential imino protons are within this distance and NOESY spectra thus provide correlation between consecutive base pairs within A-helical regions of the RNA, both to the next nucleotide within the sequence as well as across strands. Uridine and guanosine imino protons can be distinguished by their characteristic ¹⁵N chemical shifts in the ¹H,¹⁵N-TROSY experiment, where guanosine imino protons in canonical G-C base pairs have a chemical shift between 145 and 150 ppm (δ ¹⁵N) and uridine imino protons in canonical A-U base pairs between 160 and 164 ppm (δ ¹⁵N). Non-canonical U-U or G-U base-pairs result in upfield shifts of the corresponding imino proton resonances to 10-11.5 ppm/10.5-12.5 ppm (δ ¹H) and 141-145 ppm/155-160 ppm (δ ¹⁵N) for guanosine and uridine residues, respectively. Further evidence for canonical base-pairing is provided by the HNN-COSY experiment, which correlates the donor nitrogen of the guanosine or uridine imino group with the acceptor nitrogen atom of the corresponding cytidine or adenosine residue via through-space scalar ^{2h}J-NN coupling. Non-canonical U-U or G-U base-pairs do not give rise to cross peaks in HNN-COSY spectra and can thus be distinguished from involvement into canonical base pairing. For unstable A-U base pairs, for which imino resonances are broadened beyond detection due to solvent exchange, the inventors recorded long-range (lr) HNN-COSY experiments correlating the adenosine H2 proton to the uridine imino group across the hydrogen bond. The cytidine amino groups involved in base pairing can be detected in the ¹H,¹H-NOESY experiment due to their strong cross peaks to the partner guanosine imino protons and can be correlated to their respective N4 nitrogen via an exchange-optimized ¹H,¹⁵N-HSQC. The ¹H,¹⁵N-CPMG-NOESY experiment complements imino-to-amino correlations in A-form helical structural regions of RNAs. With this standard set of experiments, secondary structure predictions for the abovementioned SARS-CoV-2 RNAs were probed. Experimental secondary structures were thus determined for all 15 individual RNA elements and are described in the 'Results' section herein.

For sufficiently small RNAs (up to ~35 nt), natural abundance ¹H,¹³C HMQC experiments were recorded and analyzed to further assign aromatic H6/H8 and H5 protons and anomeric H1' ribose protons. Assignment of these NMR signals is essential for the NMR sequential assignment procedure via ¹⁵N-filtered NOESY experiments in regular A-form conformations. The ¹H,¹H-TOCSY experiment provides a quick identification of intra-nucleobase H5-H6 cross peaks for pyrimidines in this region, reducing ambiguities resulting from poor signal dispersion observed for RNAs larger than ~30 nts in general. Line shapes of these TOCSY cross peaks are also a good indication for dynamics exhibited by the respective nucleotide. The strongest signals are usually observed for highly flexible pyrimidine residues in loops. In addition, ribose H1'-H2' cross peaks can be observed for the non-A-form 2'-endo-puckered ribose conformations in the spectral region of 4-6 ppm (δ ¹H).

Guanosine H8 protons and adenosine H8 and H2 protons are correlated with N7/N9 or N1/N3 nitrogens by a long-range (lr) ¹H,¹⁵N-HSQC experiment. H2 protons of base-paired adenosines identified in the ¹H,¹H-NOESY experiment due to their strong cross peak to the partner uridine imino proton can thus be used to assign adenosine N1 and N3 atoms.

In summary, by combining reactivity profiles by DMS footprinting and extensive NMR-spectroscopic analysis of a total of 22 RNA constructs representing regulatory relevant regions of the SARS-CoV-2 genome, conclusive experimental validation of 15 RNA secondary structure models was achieved: SLs 1 to 8 at the 5'-genomic end; the attenuator hairpin and the pseudoknot of the frameshift regulating region, and SLs 1 to 3 and s2m of the 3' genomic end (see Figure 1).

### Screening of RNA targets with libraries of increasing chemical complexity

After establishing the NMR-screening platforms suited for RNA and a respective quality control check, a large number of different libraries were screened. The screening campaign yielded highly precise structure information for the development of antiviral drugs.

Initial hits identified by NMR-based screening are used to select drug-like compounds from commercially available compound archives, using machine-learning models trained on RNA targets. Experimental validation of these compounds by NMR provides a 2^{nd} and further generation of binders with improved properties and affinity.

### Synthesis of chemical moieties binding to the pseudoknot RNA of SARS-CoV-2

In the context of the present invention, the following chemical derivatives were designed based on the structure of the 2,4,6-trisubstituted pyrimidine **D01** which binds the pseudoknot RNA of SARS-CoV-2 with an affinity of 6 µM. The identification of **D01** was part of a NMR screening campaign previously performed in the group of the inventors (S. Sreeramulu, et al., Angew. Chem. Int. Ed. 2021, 60, 19191).

Aromatic residues were varied to create different hydrogen bonding patterns. The piperazine moiety was replaced by different cyclic and acyclic amines. As an example, the inventors chose *meso*-3,5-diaminopiperidin (**DAP**) which was found to be a mimetic for 2-desoxystreptamin (2-DOS) but shows simplified structural properties (Y. Zhou, et al., Antimicrob. Agents Chemother. 2005, 49, 4942). Since the 2-DOS subunit of aminoglycoside antibiotics was shown to be responsible for a sequence specific recognition of the RNA major groove, the inventors also designed derivatives which are distinguished by additional C1-spacers (amine precursor **k**) and ethylamine linkers (amine precursor **e**) to increase the flexibility of the ligands (S. Yoshizawa, D. Fourmy, R. G. Eason, J. D. Puglisi, Biochem 2002, 41, 6263).

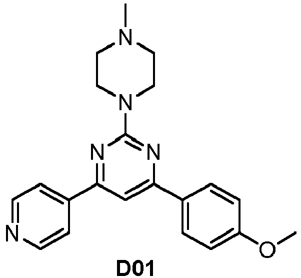

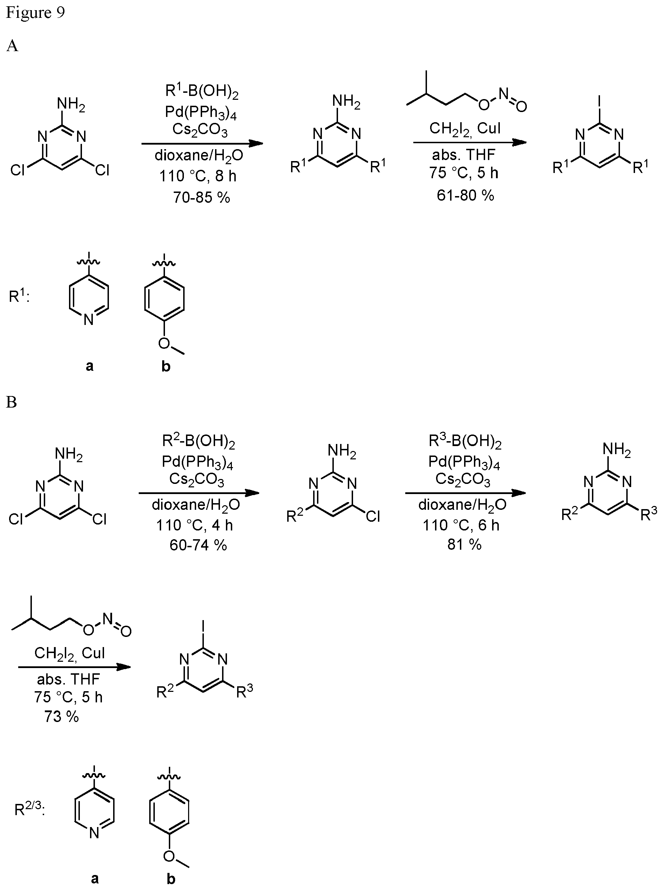

All pyrimidine precursors were synthesized via *Suzuki-Miyaura* reactions on 2-amino-4,6-dichloro-2-pyrimidine and a subsequent *Sandmeyer* type iodination (see Figure 9A and B). Asymmetrical substitution patterns were achieved through reduction of the respective boronic acid equivalents (see Figure 9B).

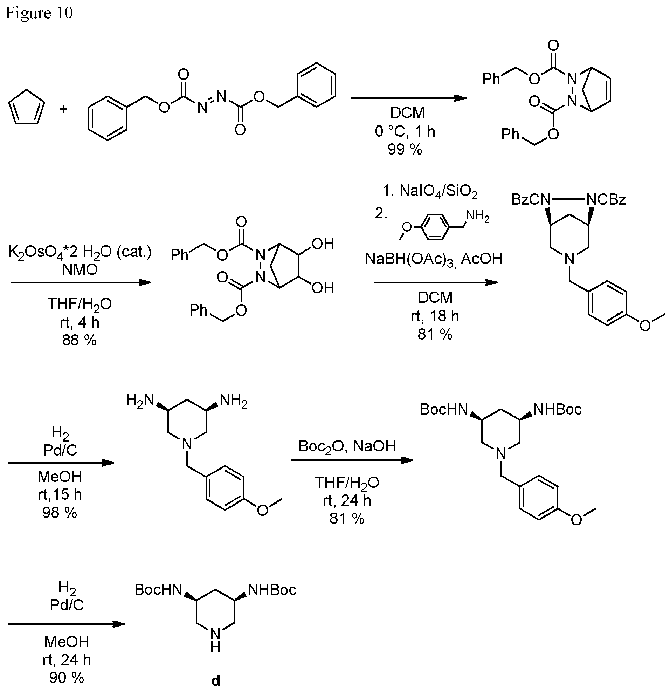

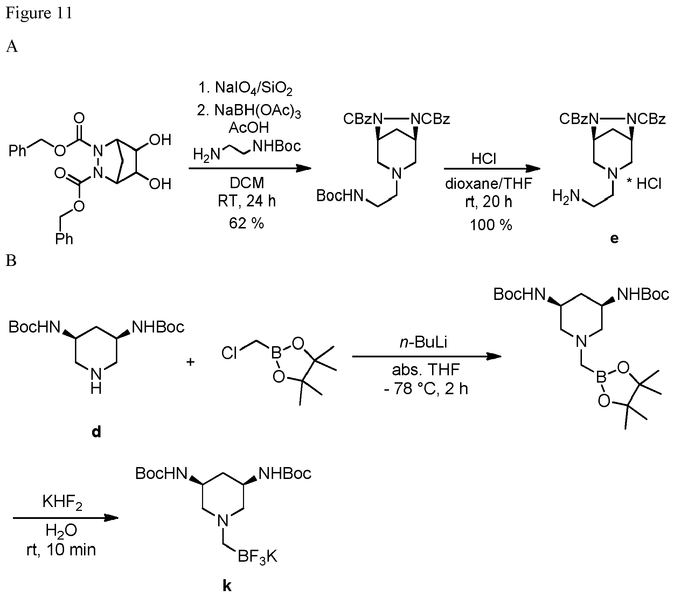

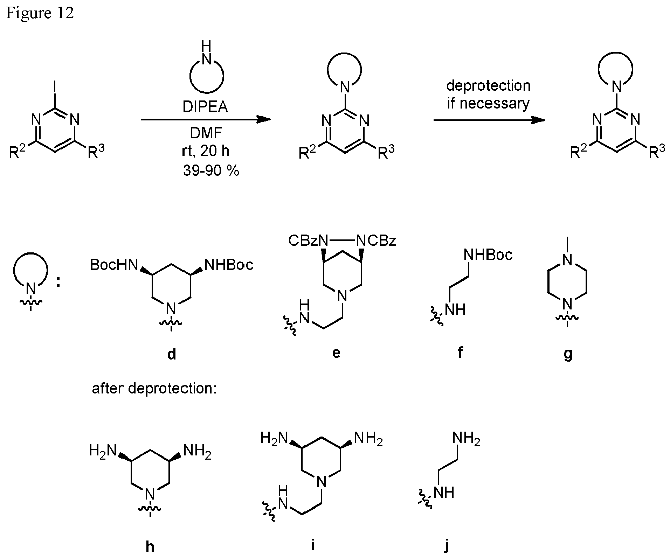

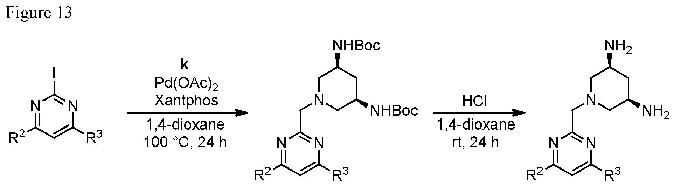

The **D01**-derivatives were synthesized via nucleophilic aromatic substitutions (see Figure 12) or *Suzuki-Miyaura* reactions (see Figure 13) between the pyrimidine precursors and different cyclic and acyclic amines. The synthesis scheme for the cyclic amine precursors **d, e, k is** shown in Figures 10 and 11.

The structures of the **D01**-derivatives according to the invention were as follows.

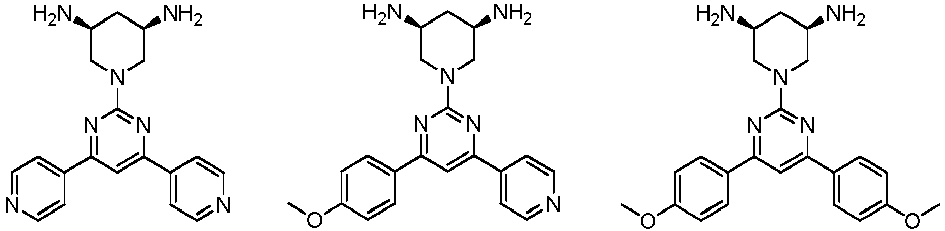

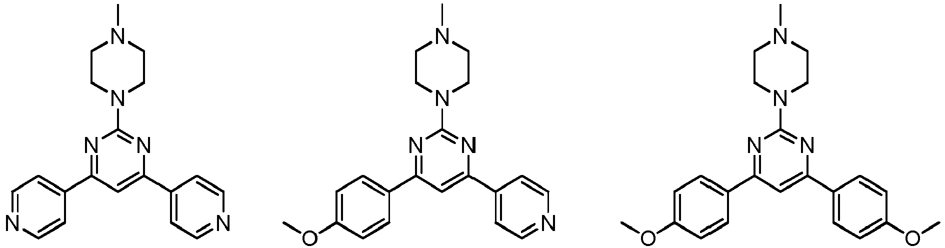

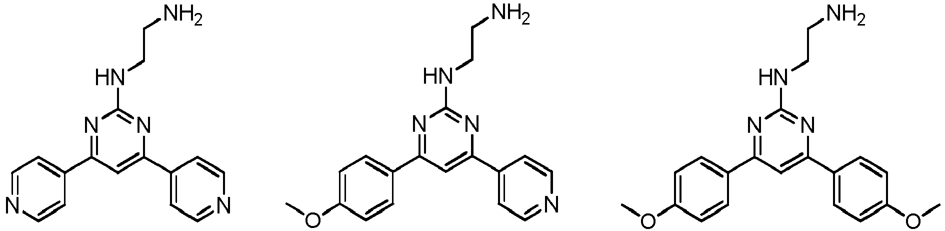

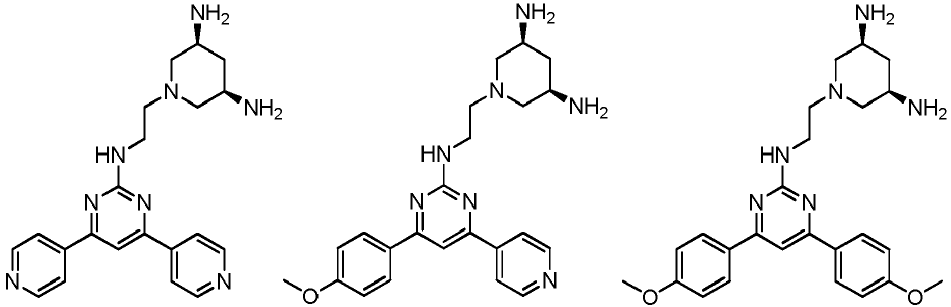

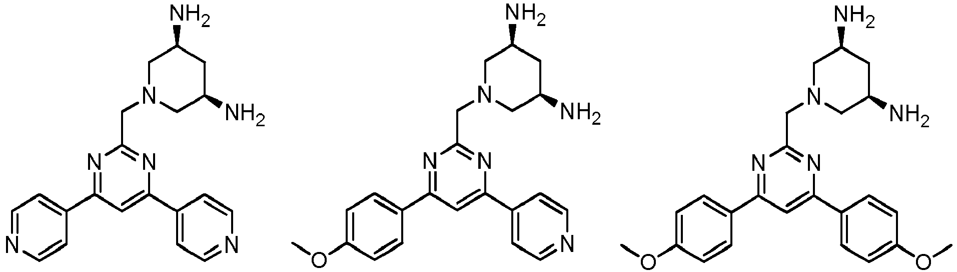

### Synthesis of asymmetrical pyrimidine precursors

### Synthesis of 4-chloro-6-(pyridin-4-yl)pyrimidin-2-amine 4a

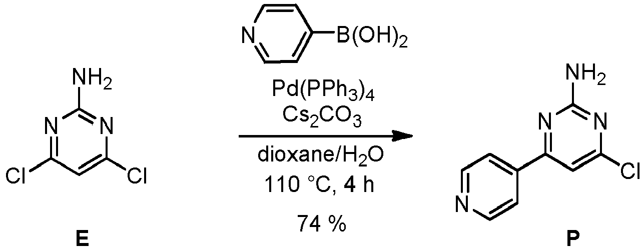

**E** (2.00 g, 12.20 mmol, 1 eq), pyridin-4-ylboronic acid (2.25 g, 18.30 mmol, 1.5 eq) and Cs₂CO₃ (19.87 g, 61.0 mmol, 5 eq) were placed into a Schlenk flask. Dioxane (135 mL) and water (15 mL) were added and the mixture was degassed with argon for 10 min. After the addition of Pd(PPh₃)₄ (282 mg, 0.24 mmol, 0.02 eq) the mixture was heated to 110 °C for 4 h. After cooling to room temperature the suspension was filtered over Celite and eluted with ethyl acetate. The aqueous phase was extracted with ethyl acetate and the combined organic layers were washed with brine and dried over MgSO₄. The extract was adsorbed on Celite and purified by flash chromatography (silica, CH₂Cl₂:MeOH 1:0 → 9:1). The product was obtained as a light yellow solid. Yield: 1.87 g (9.1 mmol, 74 %).

### Synthesis of 4-(4-methoxyphenyl)-6-(pyridin-4-yl)pyrimidin-2-amine

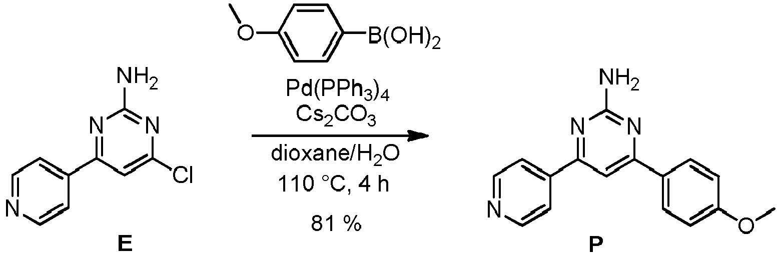

**E** (1.00 g, 4.9 mmol, 1 eq), (4-methoxyphenyl)boronic acid (1.47 g, 0.97 mmol, 2 eq) and Cs₂CO₃ (4.73 g, 14.5 mmol, 3 eq) were placed into a Schlenk flask. Dioxane (72 mL) and water (8 mL) were added and the mixture was degassed with argon for 10 min. After the addition of Pd(PPh₃)₄ (282 mg, 0.24 mmol, 0.02 eq) the mixture was heated to 110 °C for 4 h. After cooling to room temperature the suspension was filtered over Celite and eluted with ethyl acetate. The aqueous phase was extracted with ethyl acetate and the combined organic layers were washed with brine and dried over MgSO₄. The extract was adsorbed on Celite and purified by flash chromatography (silica, CH₂Cl₂:MeOH 1:0 → 9:1). The product was obtained as a light yellow solid. Yield: 1.10 g (4.0 mmol, 81 %).

### Synthesis of 2-iodo-4-(4-methoxyphenyl)-6-(pyridin-4-yl)pyrimidine

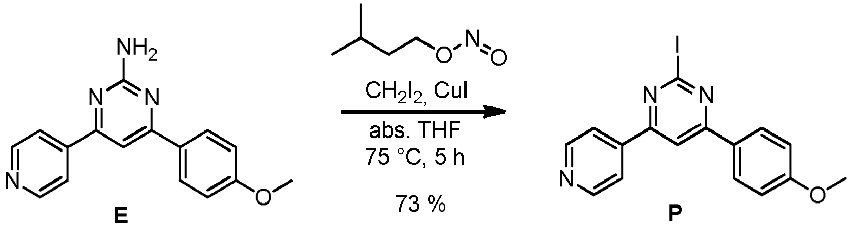

**E** (500 mg, 2.0 mmol, 1 eq), amyl nitrite (0.2 mL, 6.0 mmol, 3 eq), CH₂I₂ (0.8 mL, 10.0 mmol, 5 eq) and CuI (382 mg, 2.0 mmol, 1 eq) were placed into a Schlenk flask and solved in dry THF. The reaction mixture was heated to 75 °C for 5 h. After cooling to room temperature all volatiles were removed under reduced pressure. Ethyl acetate and water were added and the aqueous phase was extracted with ethyl acetate. The combined organic layers were washed with brine and dried over MgSO₄. The extract was adsorbed on Celite and purified by flash chromatography (silica, CH₂Cl₂:MeOH 1:0 → 9:1). The product was obtained as a light yellow solid. Yield: 530 mg (1.5 mmol, 73 %)

### Synthesis of DAP+EDA precursor e

### Synthesis of (1R,5S)-dibenzyl 3-(2-((tert-butoxycarbonyl)amino)ethyl)-3,6,7-triazabicyclo-[3.2.1]octane-6,7-dicarboxylate

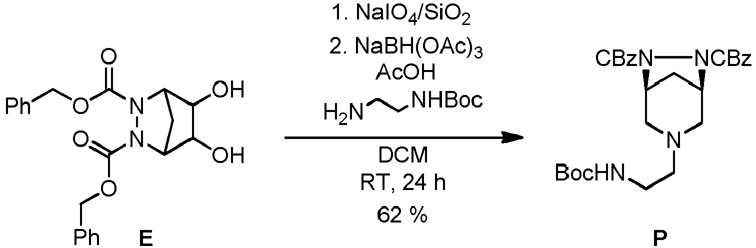

**E** (1.00 g, 2.5 mmol, 1 eq) was dissolved in 100 mL DCM and NaIO₄ on silica (10 wt. %, 6.71 g, 3.2 mmol, 1.25 eq) was added. The slurry was stirred for 4 h and filtered over MgSO₄. All volatiles were removed under reduced pressure and the residue was solved in 20 mL DCM. Tert-butyl (2-aminoethyl)carbamate (0.43 mL, 2.8 mmol, 1.1 eq), acetic acid (1 mL) and NaBH(OAc)3 (1.60 g, 7.5 mmol, 3 eq) were added and the reaction mixture was stirred for 20 h at room temperature. 100 mL saturated NaHCO₃-solution was added and the reaction mixture was extracted with DCM. The combined organic layers were dried over MgSO₄, adsorbed on Celite and purified by column chromatography (silica, cyclohexane:ethyl acetate 1:1 → 0:1) to obtain the product as a colorless oil. Yield: 821 mg (1.6 mmol, 63 %).

### Synthesis of di-tert-butyl ((3R,5S)-1-(4-(4-methoxyphenyl)-6-(pyridin-4-yl)pyrimidin-2-yl)piperidine-3,5-diyl)dicarbamate

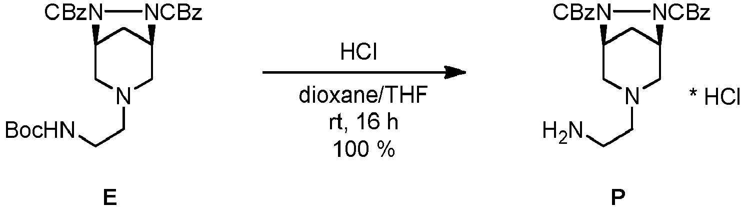

**E** (800 mg, 1.5 mmol, 1 eq) was dissolved in 10 mL THF. HCl in dioxane (5 mL, 4 M, 20.0 mmol, 13 eq) was added and the solution was stirred for 16 h. All volatiles were removed under reduced pressure. The hydrochloride salt of P was obtained as a white solid. Yield: 702 mg (1.5 mmol, 100 %).

### Synthesis of D01-derivatives via S_{N}Ar reactions

### Synthesis of di-tert-butyl ((3R,5S)-1-(4-(4-methoxyphenyl)-6-(pyridin-4-yl)pyrimidine-2-yl)piperidine-3,5-diyl)dicarbamate

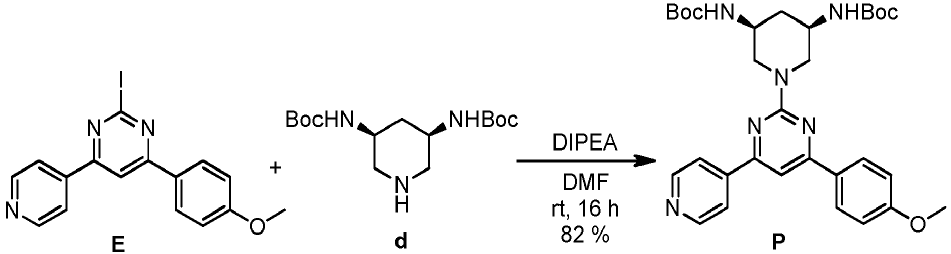

**E** (50 mg, 0.1 mmol, 1 eq) and **d** (61 mg, 0.2 mmol, 1.5 eq) were dissolved in 5 mL dry DMF. DIPEA was added and the reaction mixture was stirred for 16 h at room temperature. Brine (20 mL) was added and the mixture was extracted with ethyl acetate. The combined organic layers were washed with water and dried over MgSO₄. After the removal of all volatiles under reduced pressure the residue was purified by column chromatography (silica, DCM:MeOH:NH₃ 95:4.5:0.5). The product was obtained as a yellow solid. Yield: 61 mg (0.1 mmol, 82 %).

### Synthesis of (3R,5S)-1-(4-(4-methoxyphenyl)-6-(pyridin-4-yl)pyrimidin-2-yl)piperidine-3,5-diamine

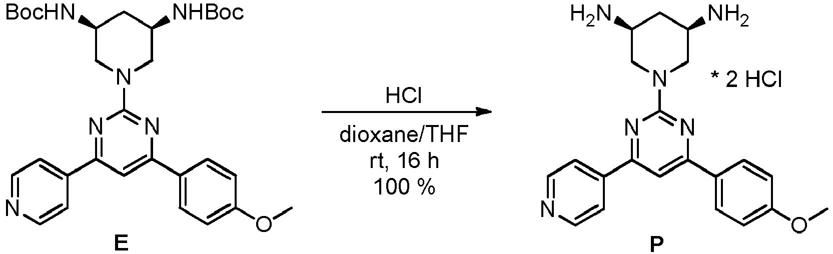

**E** (50 mg, 0.1 mmol, 1 eq) was dissolved in 2 mL THF. HCl in dioxane (0.5 mL, 4 M, 2.0 mmol, 23 eq) was added and the solution was stirred for 16 h. All volatiles were removed under reduced pressure. The hydrochloride salt of **P** was obtained as a white solid. Yield: 39 mg (0.1 mmol, 100 %).

### Synthesis of di-tert-butyl ((3S,5R)-piperidine-3,5-diyl)dicarbamate

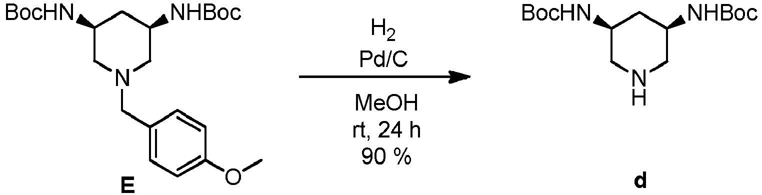

Palladium on charcoal (1.47 g, 10 wt. %, 1.4 mmol, 0.4 eq) was placed into a Schlenk flask under an argon atmosphere. **E** (1.50 g, 3.4 mmol, 1 eq) in MeOH (40 mL) was added and the flask was evacuated and filled with argon several times. Afterwards the flask was evacuated and filled with H₂. The reaction mixture was stirred at room temperature for 24 h. The catalyst was removed by a Celite filtration and the filtrate was purified by column chromatography (silica, DCM:MeOH:NH₃ 95:4.5:0.5). The product **d** was obtained as a white solid. Yield: 990 mg (3.1 mmol, 91 %).

### Binding site determination

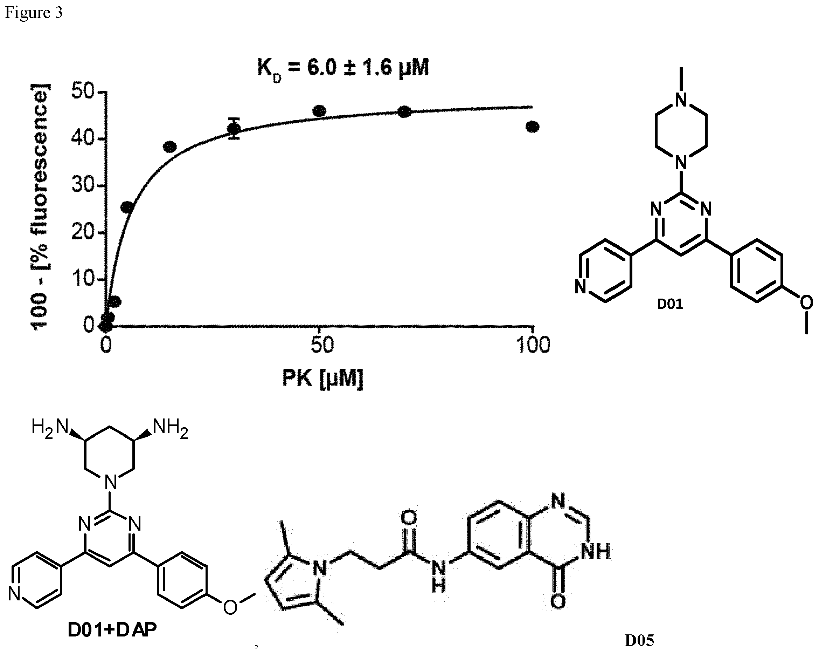

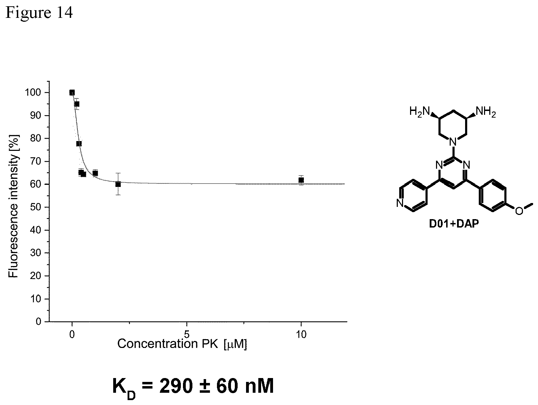

Of all previously evaluated derivatives, **D01+DAP** showed the highest binding affinity to the SARS-CoV-2 pseudoknot, which was examined via ligand observed fluorescence titrations (see Figure 3 and Figure 14). Therefore, the inventors chose this ligand for further experimental binding site determination.

For the mapping of the sequences, with respect to both experimental and *in silico* results the inventors relied on the crystal structure published by Roman *et al.* The underlying RNA construct identifies differences by a replacement of loop 2 with an AAACA pentaloop that does contain the binding site for an engineered antibody fragment to facilitate cocrystallization (C. Roman, A. Lewicka, D. Koirala, N.-S. Li, J. A. Piccirilli, ACS Chem. Biol 2021, 16, 1469).

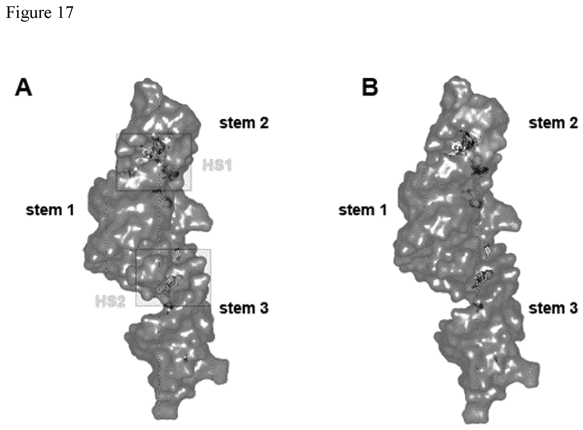

To identify potential binding sites within this three dimensional structure, the inventors used the computational mapping server FTMap. This method distributes 16 different small molecule probes on the RNA surface and finds the most favorable positions for each probe type (D. Kozakov, L. E. Grove, D. R. Hall, T. Bohnuud, S. E. Mottarella, L. Luo, B. Xia, D. Beglov, S. Vajda, Nat. Protoc. 2015, 10, 733). The results show that the three dimensional pseudoknot structure contains two separate binding hot spots (HS1 and HS2) that each contain several probe clusters (Figure 17A).

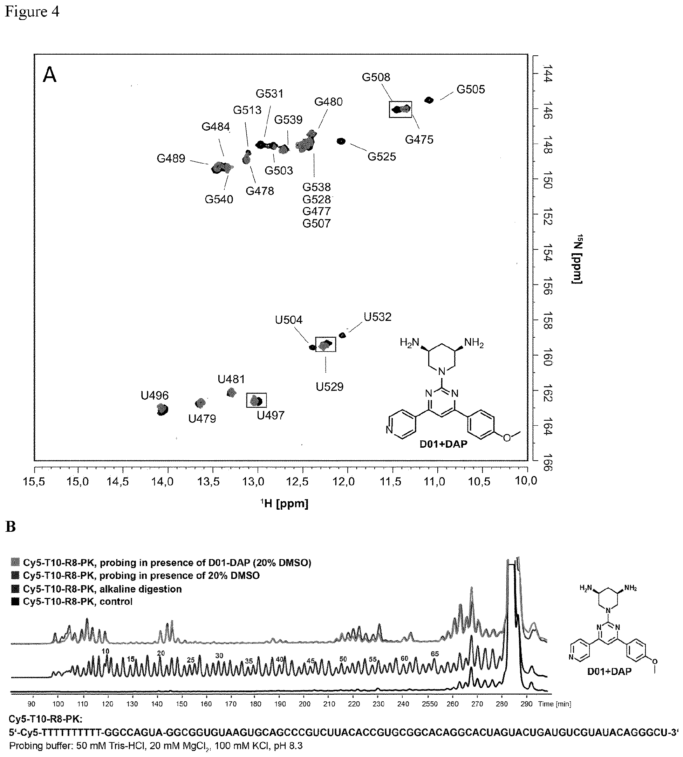

To support these results, the inventors also performed ¹H,¹⁵N-HSQC NMR experiments of the pseudoknot RNA and determined chemical shift perturbation and a decreasing signal intensity of specific signals with the addition of D01+DAP (Figure 4A). Mapping of the affected signals to the three- dimensional structure of the pseudoknot RNA confirmed one of the binding hot spots (HS2) as a binding site for D01+DAP (Figure 17A). Since under these conditions there are not enough observable signals coming from HS1, the inventors also performed inline probing experiments (Figure 4B), which indicate an interaction of D01+DAP and the pseudoknot RNA in the predicted HS1 and the loop region (L2).

### Synthesis of D05-derivatives

Similar to D01, the compound **D05** (3-(2,5-dimethyl-1H-pyrrol-1-yl)-N-(4-oxo-3,4-dihydroquinazolin-6-yl)propanamide) was derived from a DSi-poised fragment library using an in-silico optimization to fragment hits binding to the pseudknot-RNA structure of the SARS-CoV-2 frameshifting element (see Figure 14).

The compound showed interactions with the RNA in NMR- and in-line probing experiments. To specify these interactions and increase selectivity to the target RNA, the inventors divided **D05** into three structural parts: i) a non-basic heteroaromatic ring, ii) a linker and iii) a pyrimidine-like base connected via an aromatic ring to the linker.

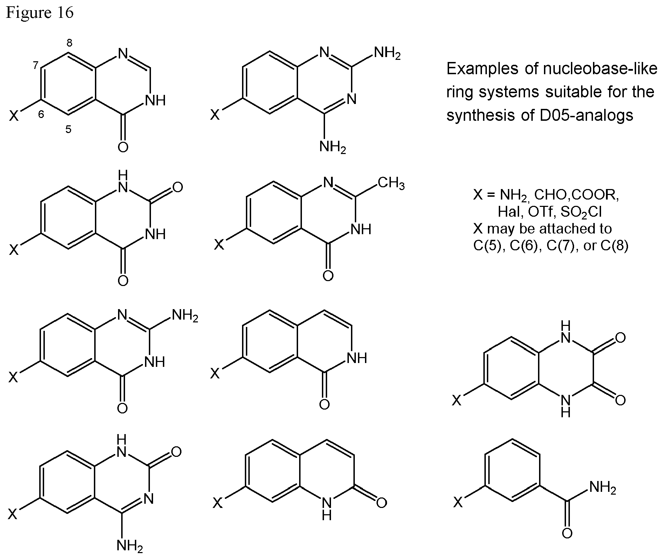

The inventors first variated the ring structure to be aromatic, heteroaromatic or aliphatic to check on stacking characteristics or other interactions for favorable binding to the target RNA. The linker was changed in length as well as in functional groups like amides or urea-like connections to the second aromatic ring. Most adjustments were performed on the second ring system where pyrimidine like nucleobases are conjugated to an unsubstituted phenyl-ring. In addition, the position to the linker was varied on the phenylic ring with different functional groups (NH₂, CHO, COOR, Hal, OTf, SO₂Cl) to optimize synthesis and modify the functional group in the linker. See also Figure 16. Examples were as follows:

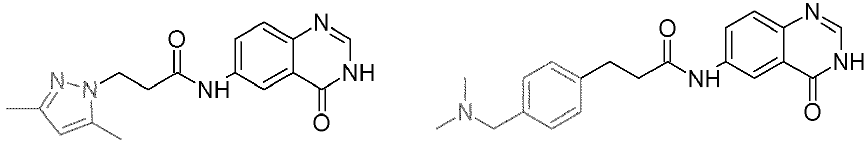

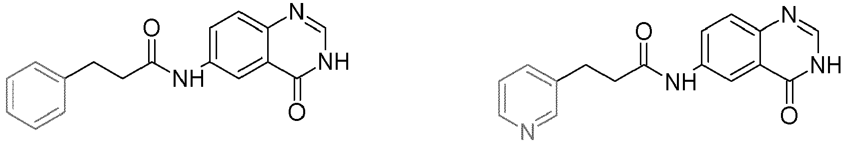

For the compounds as designed, toxicity, solubility, and microsomal stability is tested to obtain candidates for preclinical development. The lead optimization of high-affinity RNA leads ligands with confirmed anti-viral effects and clear mode-of-action.

**Biological testing**

## Claims

1. A method for identifying a pharmaceutically active compound that modulates the function of a frame-shift pseudoknot element and/or modulates the function of a translation initiation-RNA element of an RNA virus in a mammalian cell, comprising
a) contacting the frame-shift pseudoknot element and/or the translation initiation-RNA element of said RNA virus or a functional fragment thereof with at least one candidate compound, and
b) detecting a binding of the at least one candidate compound to the frame-shift pseudoknot element and/or the translation initiation-RNA element of said RNA virus or a functional fragment thereof,
wherein a binding of said candidate compound is indicative for a pharmaceutically active compound.

2. The method according to claim 1, wherein the frame-shift pseudoknot element and/or the translation initiation-RNA element furthermore includes at least one viral RNA-viral protein complex.

3. The method according to claim 1 or 2, further comprising the step of detection a modulation of the translation of one or more viral proteins of said RNA virus compared to the translation in the absence of said at least one candidate compound, wherein a modulation of the translation of the said at least one or more viral protein is indicative for a pharmaceutically active compound.

4. The method according to claim 3, wherein the translation of the one or more viral proteins of said RNA virus is essential for viral infection and/or replication.

5. The method according to claim 3 or 4, wherein the modulation leads to an increase or decrease of said translation of said at least one viral protein.

6. The method according to any one of claims 1 to 5, wherein the RNA virus is selected from the group consisting of pathogenic RNA viruses, coronaviruses, betacoronaviruses, SARS, MERS, SARS-CoV-2, NL63, OC43, and viral variants and mutants thereof.

7. The method according to any one of claims 1 to 6, wherein the pseudoknot element is selected from PK, and the translation initiation-RNA element is selected from 5_SL1, 5_SL2+3, 5_SL4, 5_SL5stem, 5_SL5a, 5_SL5b+c, 5_SL6, 5_SL7, 5_SL8, 5_SL8loop, 3_SL1, 3_SL2, 3_SL3base, 3_s2m of coronavirus, and viral variants and mutants thereof.

8. The method according to any one of claims 1 to 7, further comprising the step of preidentifying at least one conserved pseudoknot element and/or translation initiation-RNA element in the viral RNA before the step of contacting with the at least one candidate compound.

9. The method according to any one of claims 1 to 8, wherein said contacting with the at least one candidate compound is performed in vitro, in cell culture or in vivo, preferably in a nonhuman mammal, or comprises in silico molecular modeling, for example using a suitable computer program.

10. The method according to any one of claims 1 to 9, wherein the frame-shift pseudoknot element or the translation initiation-RNA element is produced by T7 polymerase-based *in-vitro* transcription or RNA synthesis methods.

11. The method according to any one of claims 1 to 10, wherein the binding is detected using ligand observed fluorescence titrations.

12. The method according to any one of claims 1 to 11, wherein said candidate compound is selected from a chemical substance, a substance selected from a peptide library, a library of small organic molecules, a combinatory library, a cell extract, in particular a plant cell extract, a "small molecular drug", a repurposable drug, a protein and/or a protein fragment, and an antibody or fragment thereof, and suitable derivatives thereof.

13. The method according to any one of claims 1 to 12, further comprising c) chemically modifying the candidate compound as identified, and repeating the method according to any one of claims 1 to 12 in order to identify a second generation candidate compound with improved modulating properties, and optionally d) repeating step c) at least once.

14. A screening system for identifying a pharmaceutically active compound that modulates the function of a frame-shift pseudoknot element and/or modulates the function of a translation initiation-RNA element of an RNA virus in a mammalian cell, comprising,
a mammalian cell comprising a viral RNA comprising a viral frame-shift pseudoknot element and/or a viral translation initiation-RNA element,
optionally at least one reporter construct to detect the translation of at least one viral protein, and
further optionally, one or more candidate compounds to be tested.

15. The screening system according to claim 14, wherein said mammalian cell is selected from a hamster or human cell.

16. A compound that modulates the function of a frame-shift pseudoknot element and/or modulates the function of a translation initiation-RNA element of an RNA virus in a mammalian cell, identified according to a method according to any one of claims 1 to 13, in particular a compound that modulates the function of a frame-shift pseudoknot element of an RNA virus in a mammalian cell according to the following Formula 1 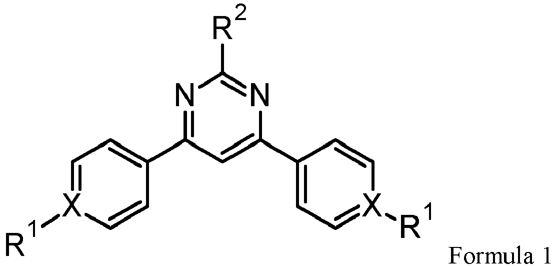
wherein X is selected from C or N, R¹ is selected from H, -OH, branched or straight C₁ to C₆ alkyl, branched or straight C₁ to C₆ alkyloxy, and -COOH, R² is selected from 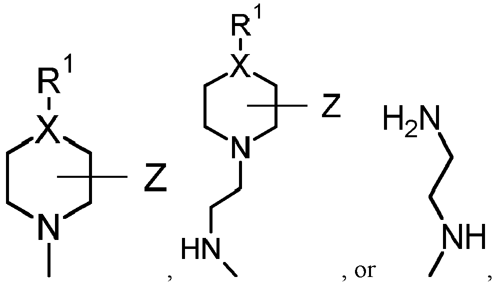
wherein X is as above, R¹ is as above, and Z indicates a mono- or disubstitution with -OH and/or -NH₂, and pharmaceutically acceptable salts and solvates thereof, or a compound that modulates the function of a frame-shift pseudoknot element of an RNA virus in a mammalian cell according to the following Formula 2 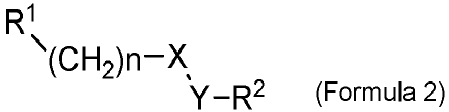
wherein n is an integer from 0 to 3, X and Y are independently selected from -NH-, -C=O-, or -CH₂- and R¹ is selected from 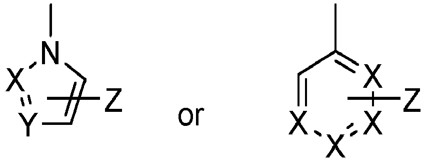
wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
and wherein R² may be attached via C(5), C(6), C(7), or C(8) of the ring structure and is selected from 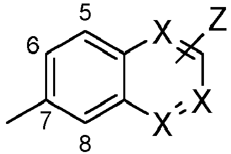
wherein X is independently selected from C or N and Z is as above, and pharmaceutically acceptable salts and solvates thereof, or a compound that modulates the function of a translation-initiation RNA element of an RNA virus in a mammalian cell according to the following Formula 3 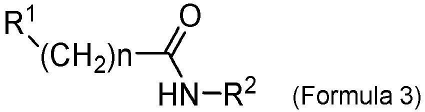
wherein n is an integer from 1 to 3, R¹ is selected from 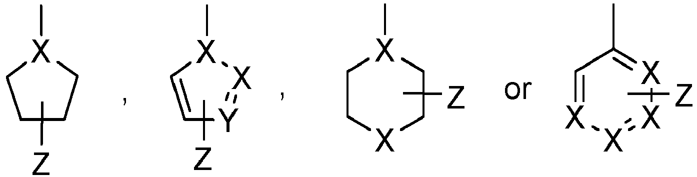
wherein X is selected from C or N, Y is selected from C, N or S and Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof
and wherein R² is selected from 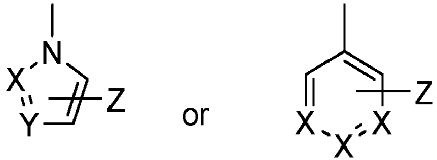
wherein X is selected from C or N and Y is selected from C, N or S, Z indicates one or multiple substitution(s) with -H, -OH, branched or straight C₁ to C₆ alkyl, branched or straight C₁ to C₆ alkyloxy, branched or straight C₁ to C₆ acyl and/or -COOH and combinations thereof, and pharmaceutically acceptable salts and solvates thereof, or a compound that modulates the function of a translation-initiation RNA element of an RNA virus in a mammalian cell according to the following Formula 4 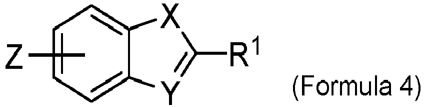
wherein X and Y are selected from C, N or S, Z indicates one or multiple substitution(s) with - H, -OH, branched or straight C₁ to C₆ acyl, branched or straight C₁ to C₆ alkyl, branched or straight C₁ to C₆ alkyloxy and/or -COOH and combinations thereof, and wherein R¹ is selected from 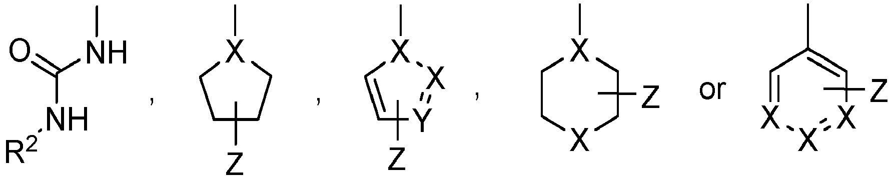
wherein R² is branched or straight C₁ to C₆ alkyl, X is selected from C or N, Y is selected from C, N or S and Z is as above, and pharmaceutically acceptable salts and solvates thereof.

17. A pharmaceutical composition comprising a compound according to claim 16, together with a pharmaceutically acceptable auxiliary agent or carrier.

18. A compound according to claim 16 or the pharmaceutical composition according to claim 17 for use in the prevention or treatment of a viral infection caused by an mRNA virus in a mammalian cell, wherein the mRNA of said mRNA virus comprises at least one of a frame-shift pseudoknot element and/or a translation initiation-RNA element.

19. The compound or the pharmaceutical composition for use according to claim 18, wherein said compound is selected from a chemical substance, a substance selected from a peptide library, a library of small organic molecules, a combinatory library, a cell extract, in particular a plant cell extract, a small molecular drug, a protein and/or a protein fragment, and an antibody or fragment thereof, and derivatives thereof.

20. The compound or the pharmaceutical composition for use according to claim 18 or 19, wherein the RNA virus is selected from the group consisting of pathogenic RNA viruses, coronaviruses, betacoronaviruses, SARS, MERS, SARS-CoV-2, HCoV-NL63, BCoV, MHV, HCoV-4408, HCoV-OC43, HCoV-HKU1, and viral variants and mutants thereof.

21. The compound or the pharmaceutical composition for use according to any one of claims 18 to 20, wherein the pseudoknot element is selected from PK, and the translation initiation-RNA element is selected from 5_SL1, 5_SL2+3, 5_SL4, 5_SL5stem, 5_SL5a, 5_SL5b+c, 5_SL6, 5_SL7, 5_SL8, 5_SL8loop, 3_SL1, 3_SL2, 3_SL3base, 3_s2m of coronavirus, and viral variants and mutants thereof.

22. A method of modulating the translation of at least one protein of an RNA virus in a mammalian cell, comprising contacting said cell with an effective amount of a compound according to claim 16 or a pharmaceutical according to claim 17.

23. A method of preventing, treating or ameliorating a disease or condition caused by a viral infection caused by an mRNA virus in a mammalian cell, wherein the mRNA of said mRNA virus comprises at least one of a frame-shift pseudoknot element and/or a translation initiation-RNA element, comprising providing an effective amount of at least one compound according to claim 16 or a pharmaceutical according to claim 17 to a patient or subject in need of said treatment, amelioration or prevention.
